# EUROPEAN PATENT APPLICATION

(11) **EP 3 536 320 A1**
(43) Date of publication of application: **11.09.2019**
(21) Application number: 17867700.1
(22) Date of filing: 02.11.2017
(51) Int. Cl.: A61K 31/496, A61P 11/00, A61P 37/06

(54) **APPLICATION OF HEDGEHOG PATHWAY INHIBITOR FOR TREATMENT OF FIBROTIC DISEASES**

(30) Priority: 04.11.2016 CN 201610972163
(71) Applicant: Impact Therapeutics, Inc, Shanghai 201210 (CN)
(72) Inventor: CAI, Sui Xiong, Shanghai 201203 (CN); TIAN, Ye Edward, Shanghai 200336 (CN)
(74) Representative: van Dam, Vincent
(86) International application number: PCT/CN2017/109021
(87) International publication number: WO 2018/082587

(57) **Abstract**

This disclosure relates to application of hedgehog pathway inhibitor in treatment for fibrosis diseases. Specially, the disclosure provides a method for treating and preventing fibrosis diseases. The method mentioned includes administering subjects in need with an effective amountof a hedgehog pathway inhibitor, whererin said hedgehog pathway inhibitor is a compound represented by the Formula (I) or a pharmaceutically acceptable salt or prodrug thereof: 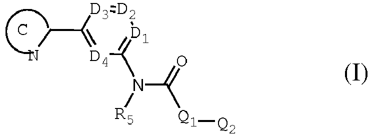 wherein C cyclic group, D₁-D₄, Q₁, Q₂, R₅ are defined herein.

## Description

### Field of the Invention

This disclosure is in the field of medicinal chemistry, and relates to treatment for fibrosis. In particular, the invention relates to application of hedegehog pathway inhibitors in treating fibrosis diseases.

### Related Art

Excessive proliferation of connective tissue leads to organ fibrosis and then loss of organ function during the repair of damaged tissues of vital organs such as liver and lung. In such cases, abnormal and/or excessive fibrous connective tissue hyperplasia is called fibrosis in those tissues that have been damaged by injury, disease or infection. Fibrosis may occur in a variety of organs. The main pathological changes are increased fibrous connective tissue in organ tissues and decreased parenchymal cells. Continuous progress may lead to organ structural damage and functional decline, or even loss, or even life threatening.

For example, all types of pulmonary fibrosis (PF) are characterized by fibroblast (Fb) proliferation and aggregation of large amounts of extracellular matrix (ECM). PF can be caused by various causes, such as occupational dust (SiO₂, etc.), radiation damage and toxicity of certain drugs (such as bleomycin). In addition, there is a class of pulmonary fibrosis with unknown etiology, called idiopathic pulmonary fibrosis (IPF). Although the causes of lesion are different, the development and outcome of PF are basically similar, that is, from the infiltration of inflammatory cells in the respiratory tract to the gradual injury and death of alveolar epithelial cells. The damaged alveolar epithelial cells release cytokines, thus activating fibroblasts, causing the migration and proliferation of fibroblasts to the damaged sites, accompanied by differentiation into myoblasts. These cells and the extracellular matrix proteins and collagen produced by them proliferate and deposit in the damaged area, further cause the injury and death of alveolar epithelial cells, form a vicious circle, and finally cause lung structure damage. Advanced PF often causes heart and lung failure and leads to death, which is of great harm to human health. At present, the exact mechanism of PF is still unclear, and no breakthrough has been made in early prevention and treatment.

In PF, IPF is the most common form of interstitial lung disease. It is a chronic and progressive malignant disease that ultimately leads to irreversible changes in the lung structure and loss of lung function. There are millions of patients worldwide (Gharaee-Kermani and Phan, 2005; Meltzer and Noble, 2008). The prognosis of IPF patients was very poor. The median survival time was only 2-3 years after diagnosis, and the 5-year survival rate was only 20% (Scotton and Chambers, 2007), which are similar to those for many malignancies. The pathology of IPF is characterized by excessive deposition and accumulation of collagen fibers and other extracellular matrix components, resulting in tissue stiffness, loss of flexibility and progressive decline in lung function. Despite more than 50 years of investigation, to date there is no efficacious therapy for IPF with lung transplantation being the only measure shown to prolong survival (Walter et al., 2006). This did not change until 2014, when FDA approved pirfenidone (Esbriet) and Ofev (nintedanib) for the treatment of IPF. Nevertheless, it is important to study and develop more effective treatments.

The occurrence of idiopathic pulmonary fibrosis is related to the abnormal activation of many cell signaling pathways, including Hedgehog signaling pathway. Hedgehog signaling pathway is an important signal pathway for early embryonic development and plays a very important role in the formation of tissue and organ morphology. For the lung, it controls the early development of the lung and the epithelial mesenchymal transformation during the formation of pulmonary branching. Unlike embryonic development, Hedgehog signaling pathways remain inactive in human tissue cells. Some fibrosis diseases, including idiopathic pulmonary fibrosis, are related to abnormal activation of Hedgehog signaling pathway in organ tissues. Therefore, inhibiting abnormal activation of Hedgehog signaling pathway and returning its activity to low or inactive state may be a method for the treatment of fibrotic diseases.

Hedgehog signaling pathway mainly includes Hedgehog protein, Hedgehog transmembrane receptor Patched-1 (PTC), transmembrane protein Smoothened (SMO), transcription factor GLI and genes in downstream control expression. Among vertebrates, three Hedgehog proteins have been found, namely Sonic Hedgehog (SHH), Desert Hedgehog (DHH) and Indian Hedgehog (IHH).

In mammalian cells, the typical Hedgehog signaling pathway occurs in primary cilia, which is organelles that are widely present on the surface of various cells and play an important sensory role in sensing changes in extracellular mechanical and chemical signals and assisting them to be transduced into cells to induce cellular responses. In the absence of Hedgehog protein, Hedgehog transmembrane receptor PTC binds and inhibits the activity of transmembrane protein SMO. As signal transducers, SMO controls the activity of members of the GLI transcription factor family. Without Hedgehog proteins such as SHH, GLI1 was not expressed, while GLI2 and GLI3 were expressed as weak and strong transcription suppressor, respectively. In the case of SHH expression, the binding of SHH with the receptor PTC results in PTC disengagement from SMO, which enables SMO to get rid of the inhibited state and activates the transcription factor to start the expression of downstream controlled genes, thus activating the whole signaling pathway.

Studies have shown that the Hedgehog signaling pathway is activated in the lung tissues of IPF patients; In vitro studies have shown that the Hedgehog-Glioma-associated oncogene homology (GLI) pathway plays an important role in the function and differentiation of fibroblasts, especially at the level of GLI transcription factor. In a bleomycin-induced mouse model of pulmonary fibrosis, the Hedgehog signaling pathway is activated, and inhibition of its expression can lead to fibrosis reduction. Meanwhile, Hedgehog signaling pathway may also be involved in a variety of signaling pathways, such as mTOR signaling pathway. However, at present, the relationship between Hedgehog signaling and other signaling pathways is not clear in the pathogenesis of pulmonary fibrosis.

Horn et al. (Ann Rheum Dis, 71: 785-789 (2012)) reported the efficacy of Hedgehog pathway inhibitor LDE223 in the treatment of bleomycin-induced skin fibrosis in Tsk-1 mice. The results showed that LDE223 prevented the skin fibrosis and resulted in the recovery of the formed fibrosis, and showed a good anti-fibrosis effect in the animal model of skin fibrosis.

Hirsova et al. (Plos One, 8(7): e70599-e70599 (2013)) reported the effect of vismodegib (GDC-0449), a hedgehog pathway inhibitor, in a foodborne non-alcoholic fatty hepatitis (NASH) model in mice. The results showed that vismodegib can reduce TRAIL-mediated liver injury in a model of NASH, thereby attenuating hepatic inflammation and fibrosis.

Moshai et al. (American Journal of Respiratory Cell and Molecular Biology, 51(1): 11-25 (2014)) reported the in vivo study of hedgehog pathway inhibitors including GDC-0449 in bleomycin-induced lung injury in a mouse model. The results suggested, although GDC-0449 showed no efficacy, hedgehog pathway inhibitor GANT61 had a good therapeutic effect on pulmonary fibrosis.

WO2014012511 disclosed compounds represented by Formula (I) as hedgehog pathway inhibitors 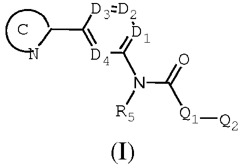
wherein: C cyclic group is an optionally substituted nitrogen-containing heteroaryl;
D₁ is N or CR₆; D₂ is N or CR₇; D₃ is N or CR₈; D₄ is N or CR₉;
Q₁ and Q₂ are independently an optionally substituted aryl, heteraryl, carbocyclic group, or heterocyclic group;
R₆-R₉ are each independently hydrogen, halo, optionally substituted amino, alkoxy, C₁₋₁₀ alkyl, C₃₋₈ cycloalkyl, haloalkyl, aryl, a carbocyclic group, a heterocyclic group, heteroaryl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, hydroxyalkoxy, aminoalkyl, aminoalkoxy, carboxyalkyl, carboxyalkoxy, nitro, cyano, acylamino, aminocarbonyl, hydroxy, thiol, acyloxy, azido, carboxy, hydroxyacylamino, alkylsulfonyl, aminosulfonyl, dialkylaminosulfonyl, alkylsulfinyl, or alkylthiol;
R₅ is hydrogen or C₁₋₁₀ alkyl, or R₅ is taken together with the N atom to which it is attached to, and other groups such as the carbon atom in C(=O) and an atom of Q₁ to form a heterocyclic ring.

In addition, WO2012159565 and WO2013013614 disclosed another compounds as hedgehog pathway inhibitors.

### SUMMARY OF THE INVENTION

The disclosure provides hedgehog pathway inhibitors for treating or preventing fibrosis diseases. In one embodiment, the hedgehog pathway inhibitors are compounds of Formula (I). In one embodiment, compounds of Formula (I) are used to treat or prevent fibrosis diseases, such as pulmonary fibrosis diseases, especially idiopathic pulmonary fibrosis, as well as liver fibrosis diseases.

The present invention also provides the use of hedgehog pathway inhibitors in the preparation of drugs for the treatment or prevention of fibrosis. In one embodiment, the hedgehog pathway inhibitors are compounds of Formula (I). In another embodiment, the fibrotic disease is pulmonary fibrosis, especially idiopathic pulmonary fibrosis. In another embodiment, the fibrotic disease is a disease of liver fibrosis.

The invention also provides a method for the treatment or prevention of fibrosis by administering an effective amount of a hedgehog pathway inhibitor to a subject in need. In one embodiment, the method includes administration of an effective amount of a compound of Formula (I). In another embodiment, the fibrotic disease is pulmonary fibrosis, especially idiopathic pulmonary fibrosis. In another embodiment, the fibrotic disease is a disease of liver fibrosis.

Specifically, the useful compounds of the present disclosure are small-molecule hedgehog pathway inhibitors. These inhibitors include, but are not limited to, the compounds described herein and, in particular, include, but are not limited to, compounds disclosed by WO2014012511, WO2012159565, and WO2013013614.

The present invention also includes the application of the hedgehog pathway inhibitors described herein in combination with other effective antifibrotic drugs to treat fibrotic diseases, including pulmonary fibrosis, hepatic fibrosis, renal fibrosis, cardiac fibrosis and scleroderma. Effective antifibrosis drugs that can be used in combination include, but are not limited to, pifenidone (Esbriet), Ofev (nintedanib), Obeticholic acid (OCA), and EDP-305, AKN-083, NP201, PXS472A, PRM-151, simtuzumab, GS-4997, GS-0976, ND-630, Cenicriviroc, FG-3019, GLPG1690 and Evogliptin.

### BRIEF DESCRIPTION OF THE FIGURES

Fig. 1 shows the changes in body weight in the prophylactic therapeutic efficacy study in unilateral pulmonary fibrosis model in SD rats.
Fig. 2 shows the percentage of changes in body weight in the prophylactic therapeutic efficacy study in unilateral pulmonary fibrosis model in SD rats.
Fig. 3 shows the gross images of left lung in the prophylactic therapeutic efficacy study in unilateral pulmonary fibrosis model in SD rats.
Fig. 4 shows the changes of lung volume in the prophylactic therapeutic efficacy study in unilateral pulmonary fibrosis model in SD rats.
Fig. 5 shows the changes of lung weight in the prophylactic therapeutic efficacy study in unilateral pulmonary fibrosis model in SD rats. Wherein, One-way ANOVA: *p<0.05 vs. Model group; T-test: #p<0.05 vs. Comp A-5mpk group.
Figure 6 shows a panoramic view of left pulmonary fibrosis lesions (HE staining) in the prophylactic therapeutic efficacy study in unilateral pulmonary fibrosis model in SD rats.

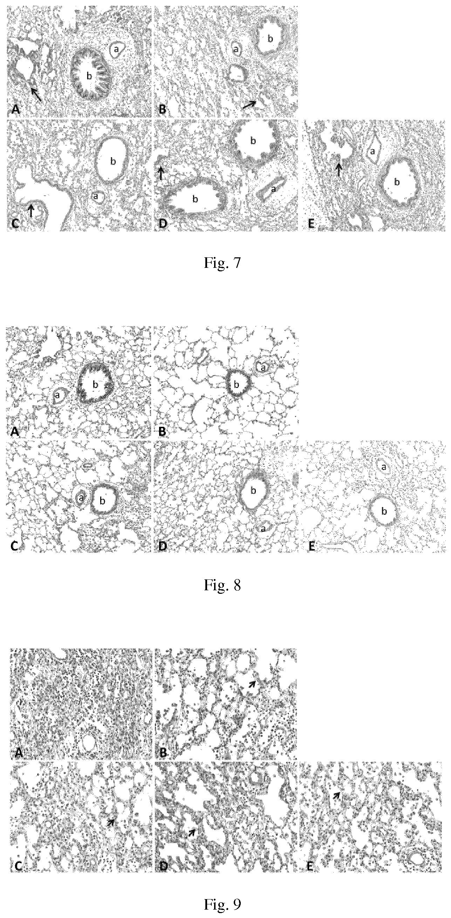

Fig. 7 shows the changes of injury of bronchioles, terminal bronchioles and pulmonary arterioles in left pulmonary fibrosis foci in the prophylactic therapeutic efficacy study in unilateral pulmonary fibrosis model in SD rats. Wherein, A: Model group, B: BIBF group, C: Comp A-5 mpk group, D: Comp A-10 mpk group, E: Comp A-20 mpk group. a: pulmonary arterioles; b: bronchioles; arrows: respiratory bronchi. Compared with Model group, fine bronchi metaplasia in the fibrosis foci, wall inflammatory infiltration and granuloma formation were significantly alleviated in each treatment group. The accompanying pulmonary arterioles injury and inflammation were also significantly reduced. In Model group, the respiratory bronchus was regenerated significantly, with various degrees of goblet cell metaplasia. Significant inhibition of regeneration was found after treatment of the test compound. H&E staining. Multiplier: 200 times.
Fig. 8 shows the changes of the peripheral bronchioles, terminal bronchioles, and pulmonary arterioles in left pulmonary fibrosis foci in the prophylactic therapeutic efficacy study in unilateral pulmonary fibrosis model in SD rats. Wherein, A: Model group, B: BIBF group, C: Comp A-5 mpk group, D: Comp A-10 mpk group, E: Comp A-20 mpk group. a: pulmonary arterioles; b: bronchioles. Compared with Model group, the injury and inflammation of the fine bronchi and the accompanying pulmonary arterioles in the fibrotic marginal region were significantly reduced. H&E staining. Multiplier: 200 times.
Fig. 9 shows the changes of alveolar tissue injury in left pulmonary fibrosis foci in the prophylactic therapeutic efficacy study in unilateral pulmonary fibrosis model in SD rats. Wherein, A: Model group, B: BIBF group, C: Comp A-5 mpk group, D: Comp A-10 mpk group, E: Comp A-20 mpk group. Arrows: Alveolar walls. The alveolar structure disappeared in Model group with a large amount of inflammatory cell infiltration and cell proliferation in the tissue. A large amount of inflammatory cell infiltration around the small artery. The structure of the alveolar tissue, the thickening of the alveolar wall, the infiltration of the inflammatory cells, and the inflammatory exudation of the alveoli were clearly showed in the treatment groups of BIBF and compound A. H&E staining. Multiplier: 200 times.
Fig. 10 shows the pathological score of injury of bronchioles, terminal bronchioles and pulmonary arterioles in left pulmonary fibrosis foci in the prophylactic therapeutic efficacy study in unilateral pulmonary fibrosis model in SD rats. Wherein, statistical analysis by One-way ANOVA: **p<0.01 vs. Model group; ***p<0.001 vs. Model group; statistical analysis by T-test: ##p<0.01 vs. CompA-5 mpk.
Fig. 11 shows the pathological score of the injury of peripheral bronchioles, the terminal bronchioles, and the pulmonary arterioles in left pulmonary fibrosis foci in the prophylactic therapeutic efficacy study in unilateral pulmonary fibrosis model in SD rats. Wherein, statistical analysis by One-way ANOVA: **p<0.01 vs. Model group; ***p<0.001 vs. Model group.
Fig. 12 shows the whole leaf scanning of left pulmonary fibrosis (Masson Trichrome Trichrome dye) in the prophylactic therapeutic efficacy stud in unilateral pulmonary fibrosis model in SD rats.
Fig. 13 shows the percentage of pulmonary fibrosis in the left lung in the prophylactic therapeutic efficacy study in unilateral pulmonary fibrosis model in SD rats.

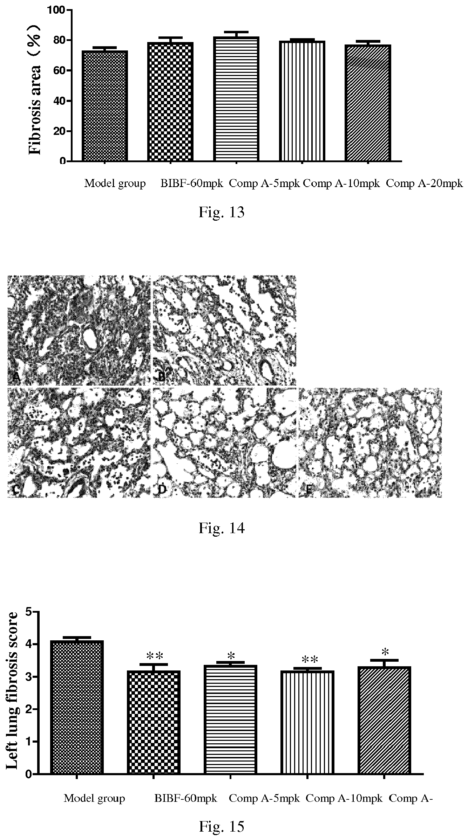

Fig. 14 shows the pathological features of left pulmonary fibrosis in the prophylactic therapeutic efficacy study in unilateral pulmonary fibrosis model in SD rats. Wherein, A: Model group, B: BIBF group, C: Comp A-5 mpk group, D: Comp A-10 mpk group, E: Comp A-20 mpk group. Arrow: alveolar wall. In Model group, the alveolar structure disappeared and a large number of fine collagen fibers were deposited in the lung tissue. The alveolar tissue structure was clearly seen in the BIBF and Comp A groups, the alveolar wall was thickened, the collagen fibers were deposited, and the inflammatory exudates in the alveolar cavity were observed. Some of them were accompanied by collagen fibers mass. Masson trichrome dyeing. Multiplier: 200 times.
Fig. 15 shows the ashcraft score of left pulmonary fibrosis pathology in the prophylactic therapeutic efficacy study in unilateral pulmonary fibrosis model in SD rats. Wherein, T-test: **p<0.01, ***p<0.001 vs. Model group.
Fig. 16 shows the ashcraft score of the degree percentage of left pulmonary fibrosis in the prophylactic therapeutic efficacy study in unilateral pulmonary fibrosis model in SD rats. Wherein, T-test: **p<0.01, ***p<0.001 vs. Model group.
Fig. 17 shows the pathological scores of bronchioles, terminal bronchioles, and pulmonary arterioles in the left pulmonary fibrosis foci in the therapeutic efficacy study in unilateral pulmonary fibrosis model in SD rats. Wherein, statistical analysis by One-way ANOVA: **p<0.01 vs. Model group; ***p<0.001 vs. Model group.
Fig. 18 shows the pathological score of peripheral bronchiole, terminal bronchiole and pulmonary arteriole injury in left pulmonary fibrosis focus in the therapeutic efficacy study in unilateral pulmonary fibrosis model in SD rats. Wherein, statistical analysis by One-way ANOVA: *p<0.05 vs. Model group.
Fig. 19 shows the percentage of area of left pulmonary fibrosis in the therapeutic efficacy study in unilateral pulmonary fibrosis model in SD rats.
Fig. 20 shows the ashcraft score of left pulmonary fibrosis in the therapeutic efficacy study in unilateral pulmonary fibrosis model in SD rats. Wherein, statistical analysis by One-way ANOVA: **p<0.01 vs. Model group; ***p<0.001 vs. Model group.
Fig. 21 shows the ashcraft score of the degree percentage of left pulmonary fibrosis in the therapeutic efficacy study in unilateral pulmonary fibrosis model in SD rats. Wherein, statistical analysis by One-way ANOVA: ***p<0.001 vs. Model group.
Fig. 22 shows the semi-quantitative analysis of immunohistochemical staining of Type I collagen in left lung in the therapeutic efficacy study in unilateral pulmonary fibrosis model in SD rats.
Fig. 23 shows the semi-quantitative analysis of immunohistochemical staining of α-SMA in left lung in the therapeutic efficacy study in unilateral pulmonary fibrosis model in SD rats. Wherein, T-test: **p<0.01 vs. Model group.
Fig. 24 shows the changes of liver weight in the prophylactic therapeutic efficacy study in liver fibrosis model in C57BL/6 mice induced by CCl₄. Wherein, T-test: ***p<0.001 vs. False Model group; ##p<0.01 vs. Model group; ###p<0.001 vs. Model group; $$$p<0.001 vs. INT747-30mpk; &p<0.05 vs. Comp A-5mpk.
Fig. 25 shows the change of ratio between liver weight and body weight in the prophylactic therapeutic efficacy study in liver fibrosis model in C57BL/6 mice induced by CCl₄. Wherein, T-test: *p<0.05 vs. False Model group; **p<0.01 vs. False Model group; ***p<0.001 vs. False Model group; ##p<0.01 vs. Model group; ###p<0.001 vs. Model group; $$$p<0.001 vs. INT747-30mpk.
Fig. 26 shows the detection value of ALT in animal peripheral blood in the prophylactic therapeutic efficacy study in liver fibrosis model in C57BL/6 mice induced by CCl₄. Wherein, T-test: *p<0.05 vs. False Model group; **p<0.01 vs. False Model group; #p<0.05 vs. Model group.
Fig. 27 shows the detection value of AST in animal peripheral blood in the prophylactic therapeutic efficacy study in liver fibrosis model in C57BL/6 mice induced by CCl₄. Wherein, T-test: *p<0.05 vs. False Model group; **p<0.01 vs. False Model group; #p<0.05 vs. Model group.
Fig. 28 shows the detection value of TBIL in animal peripheral blood in the prophylactic therapeutic efficacy study in liver fibrosis model in C57BL/6 mice induced by CCl₄. Wherein, T-test: **p<0.01 vs. False Model group; ***p<0.01 vs. False Model group; $$p<0.01 vs. INT747-30mpk.
Fig. 29 shows the pathological score of hepatocyte hydrophoretic degeneration in the prophylactic therapeutic efficacy study in liver fibrosis model in C57BL/6 mice induced by CCl₄. Wherein, T-test: *p<0.05 vs. False Model group; **p<0.01 vs. False Model group; ***p<0.001 vs. False Model group.
Fig. 30 shows the pathological score of hepatocyte necrosis in the prophylactic therapeutic efficacy study in liver fibrosis model in C57BL/6 mice induced by CCl₄. Wherein, T-test: ***p<0.001 vs. False Model group; #p<0.05 vs. Model group.
Fig. 31 shows the pathological score of hepatic inflammatory cell infiltration in the prophylactic therapeutic efficacy study in liver fibrosis model in C57BL/6 mice induced by CCl₄. Wherein, T-test: ***p<0.001 vs. False Model group; ##p<0.01 vs. Model group; $$p<0.01 vs. INT747-30mpk.
Fig. 32 shows the total score of hepatic cell injury in the prophylactic therapeutic efficacy study in liver fibrosis model in C57BL/6 mice induced by CCl₄. Wherein, T-test: **p<0.01 vs. False Model group; ***p<0.001 vs. False Model group; #p<0.05 vs. Model group.
Fig. 33 shows the pathological score of hepatic fibrosis area in the prophylactic therapeutic efficacy study in liver fibrosis model in C57BL/6 mice induced by CCl₄. Wherein, T-test: ***p<0.001 vs. False Model group; ###p<0.001 vs. Model group.
Fig. 34 shows the content of collagen in bronchoalveolar lavage fluid in the therapeutic efficacy study in lung fibrosis model in mice. Wherein, Univariate analysis of variance and Dunnett multiple comparison test were used for significance analysis: *** p<0.001 vs. Blank control group; ## p<0.01 vs. Model group, ### p<0.001 vs. Model group.
Fig. 35 shows the percentage of infiltration area of inflammatory cells in the therapeutic efficacy study in lung fibrosis model in mice. Wherein, analysis of nonparametric testing with Mann-Whitney: *** p<0.001 vs. Blank control group; # p<0.05 vs. Model group, ## p<0.01 vs. Model group, ### p<0.001 vs. Model group.
Fig. 36 shows the modified pathological Ashcroft score of pulmonary fibrosis injury in the therapeutic efficacy study in lung fibrosis model in mice. Wherein, analysis of nonparametric testing with Mann-Whitney: *** p<0.001 vs. Blank control group; ##p<0.01 vs. Model group.
Fig. 37 shows the percentage of α-SMA positive area in the therapeutic efficacy study in lung fibrosis model in mice. Wherein, analysis of nonparametric testing with Mann-Whitney: ** p<0.01 vs. Blank control group; #p<0.05 vs. Model group, ##p<0.01 vs. Model group.

### DETAILED DESCRIPTION OF THE INVENTION

In some cases, fibrosis may cause a scar formation in an affected organ, thereby destroying that functional and/or structural framework of the organ in the body. In some cases, fibrosis occurs in organs after organ transplantation and/or organ allograft transplantation. In some cases, activation of the hedgehog pathway is associated with the onset and/or progression of fibrosis as described herein.

The term "fibrosis" used herein includes, but is not limited to, pulmonary fibrosis, liver fibrosis, renal fibrosis, cardiac fibrosis and scleroderma.

Therefore, the present disclosure describes methods of inhibiting or partially inhibiting hedgehog pathway, thereby reversing or delaying the progression of fibrosis or preventing the establishment of fibrosis (for example, after organ transplantation). The present disclosure provides a method for treating or preventing warm-blooded animals, especially human beings, with or possibly suffering from fibrosis diseases, in particular for treating or preventing pulmonary fibrosis, including administering the animal an effective amount of hedgehog pathway inhibitor.

In some embodiments, the present disclosure provides a method for treating or preventing mild or moderate or severe pulmonary fibrosis, including the application of hedgehog pathway inhibitors to subjects in need.

In some embodiments, pulmonary fibrosis is idiopathic pulmonary fibrosis. In some embodiments, pulmonary fibrosis is associated with asbestosis, cystic fibrosis, infection (e.g. pneumonia), exposure to environmental allergens (e.g. coal dust, asbestos, smoking, diesel emissions, ozone, particles from industrial emissions), lung transplantation, autoimmune diseases (e.g. scleroderma), or pulmonary fibrosis is drug-induced.

In some embodiments of the above method, the application of hedgehog pathway inhibitors can reduce or reverse or decrease the progression of pulmonary fibrosis. In some embodiments, the use of hedgehog pathway inhibitors can prevent the establishment of pulmonary fibrosis.

In some embodiments, the present disclosure relates to the treatment of liver fibrosis.

In some embodiments, the liver fibrosis involved herein includes, but is not limited to, patients with chronic hepatitis B, hepatitis C, non-alcoholic steatohepatitis (NASH), alcoholic liver disease, metabolic liver disease (Wilson's disease, hemochromatosis), a bile duct obstruction (congenital or acquired) or a liver disease with an unknown cause of fibrosis.

In some embodiments of the method, hedgehog pathway inhibitors are orally administered.

In some preferred embodiments, hedgehog pathway inhibitors are the compounds represented by Formula (I) or pharmaceutically acceptable salts or prodrugs thereof 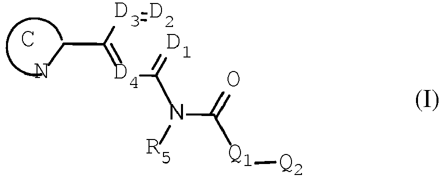
wherein: C cyclic group is an optionally substituted nitrogen-containing heteroaryl;
D₁ is N or CR₆; D₂ is N or CR₇; D₃ is N or CR₈; D₄ is N or CR₉;
Q₁ and Q₂ are independently optionally substituted aryl, heteraryl, a carbocyclic group, or a heterocyclic group;
R₆-R₉ are each independently hydrogen, halo, optionally substituted amino, alkoxy, C₁₋₁₀ alkyl, C₃₋₈ cycloalkyl, haloalkyl, aryl, a carbocyclic group, a heterocyclic group, heteroaryl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, hydroxyalkoxy, aminoalkyl, aminoalkoxy, carboxyalkyl, carboxyalkoxy, nitro, cyano, acylamino, aminocarbonyl, hydroxy, thiol, acyloxy, azido, carboxy, hydroxyacylamino, alkylsulfonyl, aminosulfonyl, dialkylaminosulfonyl, alkylsulfinyl, or alkylthiol;
R₅ is hydrogen or C₁₋₁₀ alkyl, or R₅ is taken together with the N atom to which it is attached to, and other groups such as the carbon atom in C(=O) and an atom of Q₁ to form a heterocyclic ring.

In one or more embodiments, D₁ is N or CR₆, D₂ is N or CR₇, D₃ is N or CR₈, D₄ is N or CR₉, provided that (1) D₁ is CR₆, D₂ is CR₇, D₃ is CR₈, and D₄ is CR₉, or (2) one of D₁-D₄ is N; wherein R₆-R₉ are independently selected from H, halo and C₁₋₆ alkyl.

In the preceding one or more embodiments, R₆-R₉ are each independently selected from H, halo and C₁₋₆ alkyl groups.

In one or more embodiments, the C cyclic group is an optionally substituted benzothiazolyl, pyridinyl, pyrazinyl, pyrimidinyl, pyridazinyl, indolyl, isoindolyl, 3H-indolyl, indolizinyl, indazolyl, purinyl, 4H-quinolizinyl, isoquinolyl, quinolyl, phthalzinyl, naphthyridinyl, acrindinyl, perimidinyl, phenanthrolinyl, phenazinyl, isothiazolyl, phenothiazinyl, oxazolyl, isoxazolyl, furazanyl, phenoxazinyl, 1,2-benzisoxazol-3-yl, imidazolyl, benzimidazolyl, 2-oxindolyl, thiadiazolyl, imidazo[4,5-c]pyridin-2-yl, [1,2,4]triazolo[4,3-a]pyridin-3-yl, [1,2,4]triazolo[4,3-a]pyrimidin-3-yl, [1,2,4]triazolo[4,3-a]pyrazin-3-yl, imidazo[1,2-a]pyrimidin-2-yl, imidazol[1,2-a]pyridin-2-yl, [1,2,3]triazolo[1,5-a]pyridin-2-yl or 2-oxobenzimidazolyl. In some embodiments, the substitute on the C cyclic group may be selected from the group consisting of C₁₋₄ alkyl, optionally substituted aryl, optionally substituted heteroaryl, optionally substituted heterocylic group, and optionally substituted C₃₋₈ cycloalkyl. In some embodiments, the number of substituents on the C cyclic group may be 1 or 2. Preferably, in some embodiments, when there are two substituents on the C cyclic group, one of the substitutes is C₁₋₄ alkyl.

In the preceding one or more embodiments, the C cyclic group is an optionally substituted imidazolyl, and the substitute is selected from the group consisting of C₁₋₄ alkyl, optionally substituted aryl, optionally substituted heteroaryl, optionally substituted heterocylic group, and optionally substituted C₃₋₈ cycloalkyl. In some embodiments, the C cyclic group is an optionally substituted imidazolyl, and the substitute is one of C₁₋₄ alkyl, optionally substituted aryl, optionally substituted heteroaryl, optionally substituted heterocylic group, and optionally substituted C₃₋₈ cycloalkyl. In some embodiments, the optionally substituted imidazolyl includes phenylimidazolyl, pyridinylimidazolyl, furylimidazolyl, thienylimidazolyl, thiazolylimidazolyl, pyrrolylimidazolyl, alkylimidazolyl and cycloalkylimidazolyl, wherein said phenyl, pyridinyl, furyl, thienyl, thiazolyl, pyrrolyl, alkyl and cycloakyl may be optionally substituted. In some embodiments, the substituents on said phenyl, pyridinyl, furyl, thienyl, thiazolyl, pyrrolyl, alkyl and cycloakyl are selected from C₁₋₄ alkyl and halo and the number of the substituents is 1, 2 or 3.

In the preceding one or more embodiments, the C cyclic group is an optionally substituted benzoimidazolyl, thienoimidazolyl, imidazopyridinyl or imidazothiazolyl. In some embodiments, the C cyclic group may be substituted by one or two substituents selected from C₁₋₄ alkyl and halo.

In the preceding one or more embodiments, one of D₁, D₂, D₃ and D₄ is N.

In the preceding one or more embodiments, the cyclic group containing D₁-D₄ is an optionally substituted phenyl or pyridinyl, of which the substituent is preferably alkyl, halo, haloalkyl and the like.

In the preceding one or more embodiments, Q₁ is an optionally substituted phenyl, pyridinyl or cycloalkyl.

In the preceding one or more embodiments, Q₂ is an optionally substituted phenyl, pyridinyl, pyrimidinyl, furyl, thienyl, morpholinyl, piperazinyl or piperidinyl.

In one or more embodiments, preferred compounds of Formula (I) includes but not limited to compounds represented by Formula (II): 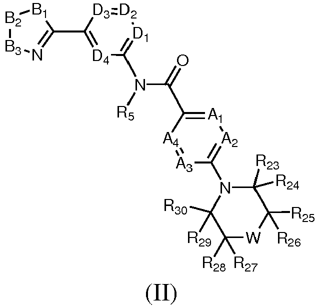 or pharmaceutically acceptable salts or prodrugs, wherein:
B₁ is NR₁₄; B₂ is CR₁₁; B₃ is CR₁₂;
R₁₁ and R₁₂ are each independently hydrogen, C₁₋₆ alkyl, C₃₋₈ cycloalkyl, thienyl and thiazolyl optionally substituted by 1, 2 or 3 groups of C₁₋₆ alkyl and halo, pyrrolyl optionally substituted by 1-4 groups of C₁₋₆ alkyl, or furyl optionally substituted by 1, 2 or 3 groups of C₁₋₆ alkyl, or, R₁₁ and R₁₂ are taken together with the C atom to which they are attached to form phenyl, pyridyl or thienyl, which is optionally substituted by 1 or 2 groups of halo and C₁₋₆ alkyl; R₁₄ is H;
D₁ is N or CR₆, D₂ is N or CR₇, D₃ is N or CR₈, D₄ is N or CR₉, provided that, (1) D₁ is CR₆, D₂ is CR₇, D₃ is CR₈, and D₄ is CR₉, or (2) one of D₁-D₄ is N; wherein, R₆-R₉ are independently H, halo and C₁₋₆ alkyl;
R₅ is H;
A₁ is N or CR₁, A₂ is N or CR₂, A₃ is N or CR₃, A₄ is N or CR₄; provided that, (1) A₁ is CR₁, A₂ is CR₂, A₃ is CR₃, and A₄ is CR₄, or (2) one of A₁-A₄ is N; wherein,R₁-R₄ are independently H, halo, C₁₋₆ alkyl or C₁₋₆ haloalkyl;
W is NR₃₃;
R₂₃, R₂₄, R₂₉ and R₃₀ are each independently H;
R₂₅ and R₂₆ are each independently H or C₁₋₆ alkyl, provided that at least one of R₂₅ and R₂₆ is C₁₋₆ alkyl;
R₂₇ and R₂₈ are each independently H and C₁₋₆ alkyl, provided that at least one of R₂₇ and R₂₈ is C₁₋₆ alkyl; and
R₃₃ is C₁₋₆ alkyl.

In the preceding one or more embodiments of Formula (II), R₁₁ and R₁₂ are each independently hydrogen, C₁₋₆ alkyl, C₃₋₈ cycloalkyl, thienyl and thiazolyl optionally substituted by 1, 2 or 3 groups of C₁₋₆ alkyl and halo, pyrrolyl optionally substituted by 1-4 groups of C₁₋₆ alkyl, or furyl optionally substituted by 1, 2 or 3 groups of C₁₋₆ alkyl. In some embodiments, one of R₁₁ and R₁₂ is H.

In the preceding one or more embodiments of Formula (II), A₁ is CR₁, A₂ is CR₂, A₃ is CR₃, and A₄ is CR₄.

In the preceding one or more embodiments of Formula (II), R₁-R₄ are independently H, halo, C₁₋₃ alkyl and C₁₋₃ haloalkyl.

In the preceding one or more embodiments of Formula (II), A₁ is CR₁, A₂ is CR₂, A₃ is CR₃, and A₄ is CR₄, R₁-R₄ are independently H, halo, C₁₋₃ alkyl and C₁₋₃ haloalkyl.

In the preceding one or more embodiments of Formula (II), R₂₆, R₂₈ and R₃₃ are independently C₁₋₃ alkyl.

In the preceding one or more embodiments of Formula (II), D₁ is CR₆, D₂ is CR₇, D₃ is CR₈, and D₄ is CR₉.

In the preceding one or more embodiments of Formula (II), R₆-R₉ are independently H, halo, or C₁₋₃ alkyl.

In the preceding one or more embodiments of Formula (II), D₁ is CR₆, D₂ is CR₇, D₃ is CR₈, and D₄ is CR₉; R₆-R₉ are independently H, halo, or C₁₋₃ alkyl.

In the preceding one or more embodiments of Formula (II), D₁ is CR₆, D₂ is CR₇, D₃ is CR₈, and D₄ is CR₉; A₁ is CR₁, A₂ is CR₂, A₃ is CR₃, and A₄ is CR₄; R₁₁ is H, C₁₋₆ alkyl or C₃₋₈ cycloalkyl, R₁₂ is C₁₋₆ alkyl or C₃₋₈ cycloalkyl; or R₁₁ is C₁₋₆ alkyl or C₃₋₈ cycloalkyl, R₁₂ is H, C₁₋₆ alkyl or C₃₋₈ cycloalkyl; R₂₆, R₂₈ and R₃₃ are each independently C₁₋₃ alkyl.

In the preceding one or more embodiments of Formula (II), B₁ is NR₁₄, B₂ is CR₁₁, B₃ is CR₁₂; R₁₁ and R₁₂ are each independently hydrogen, C₁₋₆ alkyl, C₃₋₈ cycloalkyl, thienyl and thiazolyl optionally substituted by 1, 2 or 3 groups of C₁₋₆ alkyl and halo, pyrrolyl optionally substituted by 1-4 groups of C₁₋₆ alkyl, or furyl optionally substituted by 1, 2 or 3 groups of C₁₋₆ alkyl; D₁ is CR₆, D₂ is CR₇, D₃ is CR₈, and D₄ is CR₉; A₁ is CR₁, A₂ is CR₂, A₃ is CR₃, and A₄ is CR₄. In some embodiments, one of R₁₁ and R₁₂ is H.

In the preceding one or more embodiments of Formula (II), B₁ is NR₁₄, B₂ is CR₁₁, B₃ is CR₁₂; R₁₁ and R₁₂ are each independently hydrogen or C₁₋₆ alkyl with one of R₁₁ and R₁₂ is H; D₁ is CR₆, D₂ is CR₇, D₃ is CR₈, and D₄ is CR₉; A₁ is CR₁, A₂ is CR₂, A₃ is CR₃, and A₄ is CR₄. Further, in preferred embodiments, R₆-R₉ are independently selected from H, halo or C1-3 alkyl, more preferably H or halo; R₁-R₄ are independently H, halo, C₁₋₃ alkyl and C₁₋₃ haloalkyl, more preferably H or C₁₋₃ haloalkyl.

In the preceding one or more embodiments of Formula (II), two or three of R₁-R₄ are H. In some embodiments, R₂ and R₃ are H, one of R₁ and R₄ is C₁₋₃ haloalkyl and the other one is H.

In the preceding one or more embodiments of Formula (II), two or three of R₆-R₉ are H. In some embodiments, R₈ is halo, R₆, R₇ and R₉ are H.

In the preceding one or more embodiments of Formula (II), preferred compounds of Formula (I) include, without limitation:
N-(3-(5-(thiophen-2-yl)-1H-imidazol-2-yl)-4-chlorophenyl)-2-methyl-4-((3S,5R)-3,4,5-trimethylpiperazin-1-yl)benzamide;
N-(3-(5-(thiophen-2-yl)-1H-imidazol-2-yl)-4-chlorophenyl)-2-chloro-4-((3S,5R)-3,4,5-trimethylpiperazin-1-yl)benzamide;
N-(5-(1H-benzo[d]imidazol-2-yl)-6-chloropyridin-3-yl)-6-((3S,5R)-3,4,5-trimethylpiperazin-1-yl)nicotinamide;
N-(5-(1H-benzo[d]imidazol-2-yl)-6-chloropyridin-3-yl)-2-methyl-4-((3S,5R)-3,4,5-trimethylpiperazin-1-yl)benzamide;
N-(5-(1H-benzo[d]imidazol-2-yl)-6-methylpyridin-3-yl)-2-methyl-4-((3S,5R)-3,4,5-trimethylpiperazin-1-yl)benzamide;
N-(5-(1H-benzo[d]imidazol-2-yl)-6-methylpyridin-3-yl)-6-((3S,5R)-3,4,5-trimethylpiperazin-1-yl)nicotinamide;
N-(3-(1H-benzo[d]imidazol-2-yl)-4-chlorophenyl)-2-methyl-4-((3S,5R)-3,4,5-trimethylpiperazin-1-yl)benzamide;
N-(3-(1H-benzo[d]imidazol-2-yl)-4-chlorophenyl)-2-chloro-4-((3S,5R)-3,4,5-trimethylpiperazin-1-yl)benzamide;
N-(3-(1H-benzo[d]imidazol-2-yl)-4-chlorophenyl)-2-fluoro-4-((3S,5R)-3,4,5-trimethylpiperazin-1-yl)benzamide;
N-(3-(6-chloro-1H-benzo[d]imidazol-2-yl)-4-chlorophenyl)-2-chloro-4-((3S,5R)-3,4,5-trimethylpiperazin-1-yl)benzamide;
N-(3-(6-chloro-1H-benzo[d]imidazol-2-yl)-4-chlorophenyl)-2-methyl-4-((3S,5R)-3,4,5-trimethylpiperazin-1-yl)benzamide;
N-(3-(6-fluoro-1H-benzo[d]imidazol-2-yl)-4-chlorophenyl)-2-methyl-4-((3S,5R)-3,4,5-trimethylpiperazin-1-yl)benzamide;
N-(3-(6-fluoro-1H-benzo[d]imidazol-2-yl)-4-chlorophenyl)-2-chloro-4-((3S,5R)-3,4,5-trimethylpiperazin-1-yl)benzamide;
N-(3-(1H-benzo[d]imidazol-2-yl)-4-chlorophenyl)-2-methyl-4-((3S,5R)-4-ethyl-3,5-dimethylpiperazin-1-yl)benzamide;
N-(3-(1H-benzo[d]imidazol-2-yl)-4-chlorophenyl)-2-methyl-4-((3S,5R)-4-isopropyl-3,5-dimethylpiperazin-1-yl)benzamide;
N-(3-(1H-benzo[d]imidazol-2-yl)-4-methylphenyl)-2-chloro-4-((3S,5R)-3,4,5-trimethylpiperazin-1-yl)benzamide;
N-(3-(1H-benzo[d]imidazol-2-yl)-4-chlorophenyl)-6-((3S,5R)-3,4,5-trimethylpiperazin-1-yl)nicotinamide;
N-(3-(1H-benzo[d]imidazol-2-yl)-4-chlorophenyl)-6-((3S,5R)-4-ethyl-3,5-dimethylpiperazin-1-yl)nicotinamide;
N-(3-(1H-benzo[d]imidazol-2-yl)-4-chlorophenyl)-4-methyl-6-((3S,5R)-3,4,5-trimethylpiperazin-1-yl)nicotinamide;
N-(3-(1-methyl-1H-benzo[d]imidazol-2-yl)-4-chlorophenyl)-2-chloro-4-((3S,5R)-3,4,5-trimethylpiperazin-1-yl)benzamide;
N-(3-(6-methyl-1H-benzo[d]imidazol-2-yl)-4-chlorophenyl)-2-chloro-4-((3S,5R)-3,4,5-trimethylpiperazin-1-yl)benzamide;
N-(3-(6-methyl-1H-benzo[d]imidazol-2-yl)-4-chlorophenyl)-2-methyl-4-((3S,5R)-3,4,5-trimethylpiperazin-1-yl)benzamide;
N-(3-(6-fluoro-1H-benzo[d]imidazol-2-yl)-4-chlorophenyl)-2-chloro-4-((3S,SR)-4-ethyl-3,5-dimethylpiperazin-1-yl)benzamide;
N-(3-(6-fluoro-1H-benzo[d]imidazol-2-yl)-4-methylphenyl)-2-chloro-4-((3S,5R)-3,4,5-trimethylpiperazin-1-yl)benzamide;
N-(3-(1H-benzo[d]imidazol-2-yl)-4-chlorophenyl)-2-methyl-6-((3S,5R)-3,4,5-trimethylpiperazin-1-yl)nicotinamide;
N-(3-(1H-benzo[d]imidazol-2-yl)-4-chlorophenyl)-2-chloro-6-((3S,5R)-3,4,5-trimethylpiperazin-1-yl)nicotinamide;
N-(3-(1H-benzo[d]imidazol-2-yl)-4-chlorophenyl)-4-((3S,5R)-3,4,5-trimethylpiperazin-1-yl)benzamide;
N-(3-(1H-benzo[d]imidazol-2-yl)-4-chlorophenyl)-2-trifluoromethyl-4-((3S,5R)-3,4,5-trimethylpiperazin-1-yl)benzamide;
N-(3-(1H-benzo[d]imidazol-2-yl)-4-chlorophenyl)-3-chloro-4-((3S,5R)-3,4,5-trimethylpiperazin-1-yl)benzamide;
N-(3-(1H-benzo[d]imidazol-2-yl)-4-chlorophenyl)-3-methyl-4-((3S,5R)-3,4,5-trimethylpiperazin-1-yl)benzamide;
N-(3-(1H-benzo[d]imidazol-2-yl)-4-chlorophenyl)-2-methyl-3-fluoro-4-((3S,5R)-3,4,5-trimethylpiperazin-1-yl)benzamide;
N-(3-(1H-benzo[d]imidazol-2-yl)-4-chlorophenyl)-2-chloro-5-fluoro-4-((3S,5R)-3,4,5-trimethylpiperazin-1-yl)benzamide;
N-(3-(1H-benzo[d]imidazol-2-yl)-4-chlorophenyl)-2-chloro-4-((3S,5R)-4-ethyl-3,5-dimethylpiperazin-1-yl)benzamide;
N-(3-(1H-benzo[d]imidazol-2-yl)-4-chlorophenyl)-2-chloro-4-((3S,5R)-4-isopropyl-3,5-dimethylpiperazin-1-yl)benzamide;
N-(5-(1H-benzo[d]imidazol-2-yl)-6-chloropyridin-3-yl)-2-chloro-4-((3S,5R)-3,4,5-trimethylpiperazin-1-yl)benzamide;
N-(5-(6-fluoro-1H-benzo[d]imidazol-2-yl)-6-chloropyridin-3-yl)-2-chloro-4-((3S,5R)-3,4,5-trimethylpiperazin-1-yl)benzamide;
N-(5-(6-chloro-1H-benzo[d]imidazol-2-yl)-6-chloropyridin-3-yl)-2-chloro-4-((3S,5R)-3,4,5-trimethylpiperazin-1-yl)benzamide;
N-(3-(6-fluoro-1H-benzo[d]imidazol-2-yl)-4-chlorophenyl)-2-chloro-4-((3S,5R)-3,4,5-trimethylpiperazin-1-yl)benzamide dihydrochloride;
N-(3-(1H-benzo[d]imidazol-2-yl)-4-chlorophenyl)-2-chloro-4-((3S,5R)-3,4,5-trimethylpiperazin-1-yl)benzamide dihydrochloride;
N-(3-(1H-benzo[d]imidazol-2-yl)-4-chlorophenyl)-3-chloro-4-((3S,5R)-3,4,5-trimethylpiperazin-1-yl)benzamide dihydrochloride;
N-(3-(5-(4-methylthiophen-2-yl)-1H-imidazol-2-yl)-4-chlorophenyl)-2-methyl-4-((3S,5R)-3,4,5-trimethylpiperazin-1-yl)benzamide;
N-(3-(5-(4-methylthiophen-2-yl)-1H-imidazol-2-yl)-4-chlorophenyl)-2-chloro-4-((3S,5R)-3,4,5-trimethylpiperazin-1-yl)benzamide;
N-(3-(5-(5-chlorothiophen-2-yl)-1H-imidazol-2-yl)-4-chlorophenyl)-3-chloro-4-((3S,5R)-3,4,5-trimethylpiperazin-1-yl)benzamide;
N-(3-(5-(5-chlorothiophen-2-yl)-1H-imidazol-2-yl)-4-chlorophenyl)-2-chloro-4-((3S,5R)-3,4,5-trimethylpiperazin-1-yl)benzamide;
N-(3-(5-(thiophen-3-yl)-1H-imidazol-2-yl)-4-chlorophenyl)-2-methyl-4-((3S,5R)-3,4,5-trimethylpiperazin-1-yl)benzamide;
N-(3-(5-(thiophen-3-yl)-1H-imidazol-2-yl)-4-chlorophenyl)-2-chloro-4-((3S,5R)-3,4,5-trimethylpiperazin-1-yl)benzamide;
N-(3-(5-(thiophen-3-yl)-1H-imidazol-2-yl)-4-chlorophenyl)-2-methyl-3-fluoro-4-((3S,5R)-3,4,5-trimethylpiperazin-1-yl)benzamide;
N-(3-(5-(thiophen-3-yl)-1H-imidazol-2-yl)-4-chlorophenyl)-2-chloro-5-fluoro-4-((3S,5R)-3,4,5-trimethylpiperazin-1-yl)benzamide;
N-(3-(4-methyl-5-(thiophen-2-yl)-1H-imidazol-2-yl)-4-chlorophenyl)-2-methyl-4-((3S,5R)-3,4,5-trimethylpiperazin-1-yl)benzamide;
N-(3-(5-(furan-2-yl)-1H-imidazol-2-yl)-4-chlorophenyl)-2-chloro-4-((3S,5R)-3,4,5-trimethylpiperazin-1-yl)benzamide;
N-(3-(5-(furan-2-yl)-1H-imidazol-2-yl)-4-chlorophenyl)-2-methyl-4-((3S,5R)-3,4,5-trimethylpiperazin-1-yl)benzamide;
N-(3-(5-(furan-2-yl)-1H-imidazol-2-yl)-4-chlorophenyl)-2-(trifluoromethyl)-4-((3S,5R)-3,4,5-trimethylpiperazin-1-yl)benzamide;
N-(3-(5-(furan-2-yl)-1H-imidazol-2-yl)-4-chlorophenyl)-2-methyl-3-fluoro-4-((3S,5R)-3,4,5-trimethylpiperazin-1-yl)benzamide;
N-(3-(5-(furan-2-yl)-1H-imidazol-2-yl)-4-chlorophenyl)-2-chloro-5-fluoro-4-((3S,5R)-3,4,5-trimethylpiperazin-1-yl)benzamide;
N-(3-(5-(furan-2-yl)-1H-imidazol-2-yl)-4-chlorophenyl)-2-chloro-4-((3S,5R)-4-ethyl-3,5-dimethylpiperazin-1-yl)benzamide;
N-(3-(5-(5-methylfuran-2-yl)-1H-imidazol-2-yl)-4-chlorophenyl)-2-chloro-4-((3S,5R)-3,4,5-trimethylpiperazin-1-yl)benzamide;
N-(3-(5-(5-methylfuran-2-yl)-1H-imidazol-2-yl)-4-chlorophenyl)-2-methyl-4-((3S,5R)-3,4,5-trimethylpiperazin-1-yl)benzamide;
N-(3-(5-(thiazol-2-yl)-1H-imidazol-2-yl)-4-chlorophenyl)-2-chloro-4-((3S,5R)-3,4,5-trimethylpiperazin-1-yl)benzamide;
N-(3-(5-(thiazol-2-yl)-1H-imidazol-2-yl)-4-chlorophenyl)-2-methyl-4-((3S,5R)-3,4,5-trimethylpiperazin-1-yl)benzamide;
N-(3-(5-(thiophen-2-yl)-1H-imidazol-2-yl)-4-chlorophenyl)-2-trifluoromethyl-4-((3S,5R)-3,4,5-trimethylpiperazin-1-yl)benzamide;
N-(3-(5-(thiophen-2-yl)-1H-imidazol-2-yl)-4-chlorophenyl)-2-methyl-3-fluoro-4-((3S,5R)-3,4,5-trimethylpiperazin-1-yl)benzamide;
N-(3-(5-(thiophen-2-yl)-1H-imidazol-2-yl)-4-chlorophenyl)-2-chloro-5-fluoro-4-((3S,5R)-3,4,5-trimethylpiperazin-1-yl)benzamide;
N-(3-(5-(thiophen-2-yl)-1H-imidazol-2-yl)-4-chlorophenyl)-2-chloro-4-((3S,5R)-4-ethyl-3,5-dimethylpiperazin-1-yl)benzamide;
N-(3-(5-(thiophen-2-yl)-1H-imidazol-2-yl)-4-chlorophenyl)-2-chloro-4-((3S,5R)-4-isopropyl-3,5-dimethylpiperazin-1-yl)benzamide;
N-(3-(5-(1-methyl-1H-pyrrol-2-yl)-1H-imidazol-2-yl)-4-chlorophenyl)-2-methyl-4-((3S,5R)-3,4,5-trimethylpiperazin-1-yl)benzamide;
N-(3-(5-(1-methyl-1H-pyrrol-2-yl)-1H-imidazol-2-yl)-4-chlorophenyl)-2-chloro-4-((3S,5R)-3,4,5-trimethylpiperazin-1-yl)benzamide;
N-(3-(5-(thiophen-3-yl)-1H-imidazol-2-yl)-4-chlorophenyl)-2-(trifluoromethyl)-4-((3S,5R)-3,4,5-trimethylpiperazin-1-yl)benzamide dihydrochloride;
N-(3-(5-(thiophen-3-yl)-1H-imidazol-2-yl)-4-chlorophenyl)-2-chloro-4-((3S,5R)-4-ethyl-3,5-dimethylpiperazin-1-yl)benzamide dihydrochloride;
N-(3-(1H-imidazol-2-yl)-4-chlorophenyl)-2-methyl-4-((3S,5R)-3,4,5-trimethylpiperazin-1-yl)benzamide;
N-(3-(5-ethyl-1H-imidazol-2-yl)-4-chlorophenyl)-2-(trifluoromethyl)-4-((3S,5R)-3,4,5-trimethylpiperazin-1-yl)benzamide;
N-(3-(5-ethyl-1H-imidazol-2-yl)-4-chlorophenyl)-2-methyl-3-fluoro-4-((3S,5R)-3,4,5-trimethylpiperazin-1-yl)benzamide;
N-(3-(5-ethyl-1H-imidazol-2-yl)-4-chlorophenyl)-2-chloro-5-fluoro-4-((3S,5R)-3,4,5-trimethylpiperazin-1-yl)benzamide;
N-(3-(5-ethyl-1H-imidazol-2-yl)-4-chlorophenyl)-2-chloro-4-((3S,5R)-4-ethyl-3,5-dimethylpiperazin-1-yl)benzamide;
N-(3-(5-isopropyl-1H-imidazol-2-yl)-4-chlorophenyl)-2-methyl-4-((3S,5R)-3,4,5-trimethylpiperazin-1-yl)benzamide;
N-(3-(5-isopropyl-1H-imidazol-2-yl)-4-chlorophenyl)-2-trifluoromethyl-4-((3S,5R)-3,4,5-trimethylpiperazin-1-yl)benzamide;
N-(3-(5-propyl-1H-imidazol-2-yl)-4-chlorophenyl)-2-methyl-4-((3S,5R)-3,4,5-trimethylpiperazin-1-yl)benzamide;
N-(3-(5-propyl-1H-imidazol-2-yl)-4-chlorophenyl)-2-chloro-4-((3S,5R)-3,4,5-trimethylpiperazin-1-yl)benzamide;
N-(3-(5-tert-butyl-1H-imidazol-2-yl)-4-chlorophenyl)-2-methyl-4-((3S,5R)-3,4,5-trimethylpiperazin-1-yl)benzamide;
N-(3-(5-tert-butyl-1H-imidazol-2-yl)-4-chlorophenyl)-2-chloro-4-((3S,5R)-3,4,5-trimethylpiperazin-1-yl)benzamide;
N-(3-(5-cyclopropyl-1H-imidazol-2-yl)-4-chlorophenyl)-2-chloro-4-((3S,5R)-3,4,5-trimethylpiperazin-1-yl)benzamide;
N-(3-(5-cyclopropyl-1H-imidazol-2-yl)-4-chlorophenyl)-2-methyl-4-((3S,5R)-3,4,5-trimethylpiperazin-1-yl)benzamide;
N-(3-(5-cyclopropyl-1H-imidazol-2-yl)-4-chlorophenyl)-2-(trifluoromethyl)-4-((3S,5R)-3,4,5-trimethylpiperazin-1-yl)benzamide;
N-(3-(5-cyclopropyl-1H-imidazol-2-yl)-4-chlorophenyl)-2-methyl-3-fluoro-4-((3S,5R)-3,4,5-trimethylpiperazin-1-yl)benzamide;
N-(3-(5-cyclobutyl-1H-imidazol-2-yl)-4-chlorophenyl)-2-chloro-4-((3S,5R)-3,4,5-trimethylpiperazin-1-yl)benzamide;
N-(3-(5-cyclobutyl-1H-imidazol-2-yl)-4-chlorophenyl)-2-methyl-4-((3S,5R)-3,4,5-trimethylpiperazin-1-yl)benzamide;
N-(3-(5-cyclopentyl-1H-imidazol-2-yl)-4-chlorophenyl)-2-chloro-4-((3S,5R)-3,4,5-trimethylpiperazin-1-yl)benzamide;
N-(3-(5-cyclopentyl-1H-imidazol-2-yl)-4-chlorophenyl)-2-methyl-4-((3S,5R)-3,4,5-trimethylpiperazin-1-yl)benzamide;
N-(3-(5-cyclohexyl-1H-imidazol-2-yl)-4-chlorophenyl)-2-chloro-4-((3S,5R)-3,4,5-trimethylpiperazin-1-yl)benzamide;
N-(3-(5-cyclohexyl-1H-imidazol-2-yl)-4-chlorophenyl)-2-methyl-4-((3S,5R)-3,4,5-trimethylpiperazin-1-yl)benzamide;
N-(3-(5-methyl-1H-imidazol-2-yl)-4-chlorophenyl)-2-methyl-4-((3S,5R)-3,4,5-trimethylpiperazin-1-yl)benzamide dihydrochloride;
N-(3-(5-ethyl-1H-imidazol-2-yl)-4-chlorophenyl)-2-methyl-4-((3S,5R)-3,4,5-trimethylpiperazin-1-yl)benzamide dihydrochloride;
N-(3-(5-ethyl-1H-imidazol-2-yl)-4-chlorophenyl)-2-chloro-4-((3S,5R)-3,4,5-trimethylpiperazin-1-yl)benzamide dihydrochloride;
N-(3-(5-isopropyl-1H-imidazol-2-yl)-4-chlorophenyl)-2-chloro-4-((3S,5R)-3,4,5-trimethylpiperazin-1-yl)benzamide dihydrochloride;
N-(3-(5-isopropyl-1H-imidazol-2-yl)-4-chlorophenyl)-2-methyl-3-fluoro-4-((3S,5R)-3,4,5-trimethylpiperazin-1-yl)benzamide dihydrochloride;
N-(3-(5-isopropyl-1H-imidazol-2-yl)-4-chlorophenyl)-2-chloro-5-fluoro-4-((3S,5R)-3,4,5-trimethylpiperazin-1-yl)benzamide dihydrochloride;
N-(3-(5-isopropyl-1H-imidazol-2-yl)-4-chlorophenyl)-2-chloro-4-((3S,5R)-4-ethyl-3,5-dimethylpiperazin-1-yl)benzamide dihydrochloride;
N-(3-(5-cyclopropyl-1H-imidazol-2-yl)-4-chlorophenyl)-2-chloro-5-fluoro-4-((3S,5R)-3,4,5-trimethylpiperazin-1-yl)benzamide dihydrochloride;
N-(3-(5-cyclopropyl-1H-imidazol-2-yl)-4-chlorophenyl)-2-chloro-4-((3S,5R)-4-ethyl-3,5-dimethylpiperazin-1-yl)benzamide dihydrochloride;
N-(3-(3H-imidazo[4,5-b]pyridin-2-yl)-4-chlorophenyl)-2-chloro-4-((3S,5R)-3,4,5-trimethylpiperazin-1-yl)benzamide;
N-(3-(3H-imidazo[4,5-c]pyridin-2-yl)-4-chlorophenyl)-2-chloro-4-((3S,5R)-3,4,5-trimethylpiperazin-1-yl)benzamide;
N-(3-(3H-imidazo[4,5-c]pyridin-2-yl)-4-chlorophenyl)-2-methyl-4-((3S,5R)-3,4,5-trimethylpiperazin-1-yl)benzamide;
N-(3-(1H-thieno[3,4-d]imidazol-2-yl)-4-chlorophenyl)-2-methyl-4-((3S,5R)-3,4,5-trimethylpiperazin-1-yl)benzamide;
N-(3-(1H-thieno[3,4-d]imidazol-2-yl)-4-chlorophenyl)-2-chloro-4-((3S,5R)-3,4,5-trimethylpiperazin-1-yl)benzamide;
N-(3-(1H-thieno[3,4-d]imidazol-2-yl)-4-chlorophenyl)-2-(trifluoromethyl)-4-((3S,5R)-3,4,5-trimethylpiperazin-1-yl)benzamide;
N-(3-(1H-thieno[3,4-d]imidazol-2-yl)-4-chlorophenyl)-2-methyl-3-fluoro-4-((3S,5R)-3,4,5-trimethylpiperazin-1-yl)benzamide;
N-(3-(1H-thieno[3,4-d]imidazol-2-yl)-4-chlorophenyl)-2-chloro-5-fluoro-4-((3S,5R)-3,4,5-trimethylpiperazin-1-yl)benzamide;
N-(3-(1H-thieno[3,4-d]imidazol-2-yl)-4-chlorophenyl)-2-chloro-4-((3S,5R)-4-ethyl-3,5-dimethylpiperazin-1-yl)benzamide;
and pharmaceutically acceptable salts or prodrugs thereof.

With respect to the hedgehog pathway inhibitors as mentioned herein, useful alkyl groups include straight-chained or branched C₁₋₁₀ alkyl groups, preferably straight-chained or branched C₁₋₆ alkyl groups, more preferably C₁₋₃ alkyl groups. Typical C₁₋₁₀ alkyl groups include methyl, ethyl, propyl, isopropyl, butyl, sec-butyl, tert-butyl, 3-pentyl, hexyl and octyl groups.

Preferred alkenyl group is C₂₋₄ alkenyl. Typical alkenyl groups include ethenyl, 1-propenyl, 2-methyl-1-propenyl, 1-butenyl and 2-butenyl.

Preferred alkynyl group is C₂₋₄ alkynyl. Typical alkynyl groups include ethynyl, 1-propynyl, 1-methyl-2-propynyl, 2-propynyl, 1-butynyl and 2-butynyl.

Useful alkoxy groups include oxy substituted by alkyl, e.g., one of the C₁₋₁₀ alkyl groups mentioned above, preferably the C₁₋₆ alkyl groups mentioned above (that is C₁₋₆ alkoxy), more preferably C₁₋₃ alkoxy.

Useful alkylthio groups include thio substituted by any one of the C₁₋₁₀ alkyl groups mentioned above, preferably C₁₋₆ alkyl, more preferably C₁₋₃ alkyl, which may be optionally substituted. Also included are the sulfoxides and sulfones of such alkylthio groups.

Useful amino groups include -NH₂, -NHR' and -NR'R", wherein R' and R" are C₁₋₁₀ alkyl or C₃₋₈ cycloalkyl; or R' and R" are combined with the N to form a ring structure, such as a piperidyl; or R' and R'' are combined with the N and another group to form a ring, such as a piperazinyl, which are optionally substituted.

Alkyl, alkoxy, alkylthio, alkenyl, alkynyl, cycloalkyl, carbocyclic and heterocyclic groups, may be optionally substituted by one or more (such as 1, 2, 3, or 4) substituents selected from the group consisting of halo, hydroxy, carboxy, amino, amido, nitro, cyano, C₁-C₆ acylamino, C₁-C₆ acyloxy, C₁-C₆ alkoxy, aryloxy, alkylthio, C₆-C₁₀ aryl, C₄-C₇ cycloalkyl, C₂-C₆ alkenyl, C₆-C₁₀ aryl(C₂-C₆)alkenyl, C₆-C₁₀ aryl(C₂-C₆)alkynyl, saturated and unsaturated heterocyclic and heteroaryl. In one preferred embodiment, alkoxy may be substituted by one or more (such as 1-4 or 1-3) substituents selected from the group consisting of halo, morpholino, amino including alkylamino and dialkylamino, and carboxylic ester.

Optional substituents on the aryl, arylalkyl, arylalkenyl, arylalkynyl, heteroaryl and heteroarylalkyl groups may be one or more (such as 1, 2, 3, or 4) groups selected from the group consisting of C₁-C₆ alkyl, acyl, halo, methylenedioxy, C₁-C₆ haloalkyl, C₆-C₁₀ aryl, C₄-C₇ cycloalkyl, C₂-C₆ alkenyl, C₂-C₆ alkynyl, C₆-C₁₀ aryl(C₁-C₆)alkyl, C₆-C₁₀ aryl(C₂-C₆)alkenyl, C₆-C₁₀ aryl(C₂-C₆)alkynyl, C₁-C₆ hydroxyalkyl, nitro, amino, ureido, cyano, C₁-C₆ acylamino, hydroxy, thiol, C₁-C₆ acyloxy, aminocarbonyl, azido, C₁-C₆ alkoxy, carboxy, di(Ci-Cio alkyl)amino, alkylsulfonyl, arylsulfonyl, dialkylaminosulfonyl, or alkylsulfinyl.

Useful aryl groups include C₆₋₁₄ aryl, preferably C₆₋₁₀ aryl. Typical C₆₋₁₄ aryl groups include phenyl, naphthyl, phenanthrenyl, anthracenyl, indenyl, biphenyl, biphenylene and fluorenyl groups.

Useful cycloalkyl groups are C₃₋₈ cycloalkyl. Typical cycloalkyl groups include cyclopropyl, cyclobutyl, cyclopentyl, cyclohexyl and cycloheptyl.

Useful partially saturated carbocyclic groups include cycloalkenyl groups, such as cyclopentenyl, cycloheptenyl and cyclooctenyl.

Useful halo or halogen groups include fluorine, chlorine, bromine and iodine.

Arylalkyl groups are preferably C₁₋₄ alkyl groups. Preferably the arylalkyl group is benzyl, phenethyl or naphthylmethyl.

Preferably the arylalkenyl groups are C₂₋₄ alkenyl groups.

Preferably the arylalkynyl groups are C₂₋₄ alkynyl groups.

Useful aryloxy groups include phenoxy and 4-methylphenoxy.

Useful arylalkoxy groups include benzyloxy and phenethyloxy.

Useful haloalkyl groups include C₁₋₁₀ alkyl substituted by one or more fluorine, chlorine, bromine or iodine atoms, e.g., fluoromethyl, difluoromethyl, trifluoromethyl, pentafluoroethyl, 1,1-difluoroethyl, chloromethyl, chlorofluoromethyl and trichloromethyl groups.

Useful acyl groups include, for example, C₁₋₆ acyl, e.g., formacyl, acetyl, and the like.

Useful acylamino (acylamido) groups are any C₁₋₆ acyl (alkanoyl) attached to an amino nitrogen, e.g., acetamido, chloroacetamido, propionamido, butanoylamido, pentanoylamido and hexanoylamido, as well as aryl-substituted C₁₋₆ acylamino groups, e.g., benzoylamido.

Useful acyloxy groups are any C₁₋₆ acyl (alkanoyl) attached to an oxy (-O-) group, e.g., formyloxy, acetoxy, propionoyloxy, butanoyloxy, pentanoyloxy and hexanoyloxy.

Useful saturated or partially saturated heterocyclic groups include tetrahydrofuranyl, pyranyl, piperidinyl, piperazinyl, pyrrolidinyl, imidazolidinyl, imidazolinyl, indolinyl, isoindolinyl, quinuclidinyl, morpholinyl, isochromanyl, chromanyl, pyrazolidinyl, and pyrazolinyl. A heterocyclic group also includes heteroaryl herein.

Useful heteroaryl groups include quinazolinyl, thienyl, benzo[b]thienyl, benzo[2,3-b]thienyl, thianthrenyl, furyl (furanyl), pyranyl, isobenzopyranyl, chromenyl, xanthenyl, phenoxanthiinyl, pyrrolyl, imidazolyl, pyrazolyl, pyridyl (pyridinyl, including without limitation 2-pyridyl, 3-pyridyl, and 4-pyridyl), pyrazinyl, pyrimidinyl, pyridazinyl, indolizinyl, isoindolyl, 3H-indolyl, indazolyl, purinyl, 4H-quinolizinyl, isoquinolyl, dihydroisoquinolyl, quinolyl, phthalzinyl, naphthyridinyl, acridinyl, perimidinyl, phenanthrolinyl, phenazinyl, isothiazolyl, phenothiazinyl, isoxazolyl, furazanyl, phenoxazinyl, 1,4-dihydroquinoxaline-2,3-dione, 7-aminoisocoumarin, pyrido[1,2-a]pyrimidin-4-one, tetrahydrocyclopenta[c]pyrazol-3-yl, imidazo[1,5-a]pyrimidinyl, imidazo[4,5-c]pyridin-2-yl, [1,2,4]triazolo[4,3-a]pyridin-3-yl, [1,2,4]triazolo[4,3-a]pyrimidin-3-yl, [1,2,4]triazolo[4,3-a]pyrazin-3-yl, imidazo[1,2-a]pyrimidin-2-yl, imidazo[1,2-a]pyridin-2-yl, benzo[e][1,3]oxazinyl, 1,2-benzoisoxazol-3-yl, benzimidazolyl, 2-oxindolyl, thiadiazolyl, and 2-oxobenzimidazolyl. Where the heteroaryl group contains a nitrogen atom in a ring, such nitrogen atom may be in the form of an N-oxide, e.g., a pyridyl N-oxide, pyrazinyl N-oxide and pyrimidinyl N-oxide.

Useful heteroaryloxy groups include pyridyloxy, pyrazinyloxy, pyrrolyloxy, pyrazolyloxy, imidazolyloxy and thiophenyloxy.

It is understood that the present disclosure includes any combination of the characteristics described in the above embodiments; it also includes any combination of different groups selected in the above group definition according to the definition of formula (I); it also includes any combination of groups at different positions of each specific compound listed in the invention.

Some of the compounds of the present disclosure may exist as stereoisomers including optical isomers. The disclosure includes all stereoisomers and both the racemic mixtures of such stereoisomers as well as the individual enantiomers that may be separated according to methods that are well known to those of ordinary skill in the art.

Examples of pharmaceutically acceptable salts include inorganic and organic acid salts, such as hydrochloride, hydrobromide, sulphate, citrate, lactate, tartrate, maleate, fumarate, mandelate and oxalate; and inorganic and organic base salts with bases, such as sodium hydroxy, tris(hydroxymethyl)aminomethane (TRIS, tromethane) and N-methylglucamine.

Examples of prodrugs of the compounds of the disclosure include the simple esters of carboxylic acid containing compounds (e.g., those obtained by condensation with a C_{1- 4} alcohol according to methods known in the art); esters of hydroxy containing compounds (e.g., those obtained by condensation with a C₁₋₄ carboxylic acid, C₃₋₆ diacid or anhydride thereof, such as succinic and fumaric anhydrides according to methods known in the art); imines of amino containing compounds (e.g., those obtained by condensation with a C₁₋₄ aldehyde or ketone according to methods known in the art); carbamate of amino containing compounds, such as those described by Leu, et. al., (J. Med. Chem. 42: 3623-3628 (1999)) and Greenwald, et al., (J. Med. Chem. 42: 3657-3667 (1999)); and acetals and ketals of alcohol containing compounds (e.g., those obtained by condensation with chloromethyl methyl ether or chloromethyl ethyl ether according to methods known in the art).

The hedgehog pathway inhibitor of the disclosure may be obtained using a method known to the skilled in the art or a method of reference (including patent, patent application and public case) cited by the invention, including a synthesis method disclosed by WO2014012511, WO2012159565 and WO2013013614.

The hedgehog pathway inhibitor of the disclosure may be applied in pharmaceutical compositions comprising pharmaceutical carrier, wherein the pharmaceutical compositions within the scope of this disclosure include all compositions wherein the compounds of the present disclosure are contained in an amount that is effective to achieve its intended purpose. While individual needs vary, determination of optimal ranges of effective amounts of each component is within the skilled of the art. Typically, the compounds or pharmaceutically acceptable salt thereof may be administered to mammals (for example, human), orally at a dose of 0.0025 to 50 mg/kg of body weight, per day. Preferably, approximately 0.01 to approximately 10 mg/kg of body weight is orally administered. The dose for intramuscular injection is half of the dose for oral administration, for example, approximately 0.0025 to approximately 25 mg/kg of body weight, more preferably, approximately 0.01 to approximately 5 mg/kg of body weight.

The unit oral dose may comprise from approximately 0.01 to approximately 50 mg, preferably approximately 0.1 to approximately 10 mg of the compound of the disclosure. The unit dose may be administered one or more times daily, as one or more tablets, each containing from approximately 0.1 to approximately 10 mg, conveniently approximately 0.25 to 50 mg of the compound or its solvates.

In a topical formulation, the compound may be present at a concentration of approximately 0.01 to 100 mg per gram of carrier.

When used in veterinarians, higher doses are given on demand.

Suitable pharmaceutically acceptable carriers comprise excipients and auxiliaries, which facilitate processing of the compounds into preparations that may be used pharmaceutically. Preferably, the preparations, particularly those preparations which may be administered orally and that may be used for the preferred type of administration, such as tablets, dragees, and capsules, oral cleaning agent, hair gel, shampoo, and formulations suitable for rectal administration, such as enemas and suppositories, as well as suitable solutions for administration by injection or orally, contain from approximately 0.01 to 99 percent, preferably from approximately 0.25 to 75 percent of active compound(s), together with the excipient.

Also included within the scope of the present disclosure are the non-toxic pharmaceutically acceptable salts of the compounds of the present invention. Acid addition salts are formed by mixing a solution of the hedgehog pathway inhibitor of the present disclosure with a solution of a pharmaceutically acceptable non-toxic acid, such as hydrochloric acid, fumaric acid, maleic acid, succinic acid, acetic acid, citric acid, tartaric acid, carbonic acid, phosphoric acid, oxalic acid, and the like. Base addition salts are formed by mixing a solution of the hedgehog pathway inhibitor of the present disclosure with a solution of a pharmaceutically acceptable non-toxic base, such as sodium hydroxide, potassium hydroxide, choline hydroxide, sodium carbonate, Tris, N-methylglucamine and the like.

The hedgehog pathway inhibitor of the disclosure may be administered to any mammal, as long as they may experience the beneficial effects of the compounds of the disclosure. Foremost among such mammals are humans and veterinary animals, although the disclosure is not intended to be so limited.

The hedgehog pathway inhibitor and pharmaceutical compositions of the present disclosure may be administered by any means that achieve their intended purpose. For example, administration may be by parenteral, subcutaneous, intravenous, intramuscular, intraperitoneal, transdermal, buccal, intrathecal, intracranial, intranasal or topical routes. Alternatively, or concurrently, administration may be by the oral route. The dosage administered will be dependent upon the age, health, and weight of the recipient, kind of concurrent treatment, frequency of treatment, and the nature of the effect desired.

The pharmaceutical preparations of the present disclosure are manufactured in a manner, which is itself known, e.g., by means of conventional mixing, granulating, dragee-making, dissolving, or lyophilizing processes. Thus, pharmaceutical preparations for oral use may be obtained by combining the active compounds with solid excipients, optionally grinding the resulting mixture and processing the mixture of granules, after adding suitable auxiliaries, if desired or necessary, to obtain tablets or dragee cores.

Suitable excipients are, in particular fillers, such as saccharides, e.g. lactose or sucrose, mannitol or sorbitol; cellulose preparations or calcium phosphates, e.g. tricalcium phosphate or calcium hydrogen phosphate; as well as binders, such as starch paste, using, e.g., maize starch, wheat starch, rice starch, potato starch, gelatin, tragacanth, methyl cellulose, hydroxypropylmethylcellulose, sodium carboxymethylcellulose, or polyvinyl pyrrolidone. If desired, disintegrating agents may be added, such as the above-mentioned starches and also carboxymethyl-starch, cross-linked polyvinyl pyrrolidone, agar, or alginic acid or a salt thereof, such as sodium alginate. Auxiliaries are, above all, flow-regulating agents and lubricants, e.g., silica, talc, stearic acid or salts thereof, such as magnesium stearate, calcium stearate, stearic acid or polyethylene glycol. Dragee cores are provided with suitable coatings which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices, solutions of suitable cellulose preparations, such as acetylcellulose phthalate or hydroxypropylmethyl-cellulose phthalate, are used. Dye stuffs or pigments may be added to the tablets or dragee coatings, e.g., for identification or in order to characterize combinations of active compound doses.

Other pharmaceutical preparations, which may be used orally, include push-fit capsules made of gelatin, as well as soft, sealed capsules made of gelatin and a plasticizer, such as glycerol or sorbitol. The push-fit capsules may contain the active compounds in the form of granules, which may be mixed with fillers, such as lactose; binders, such as starches; and/or lubricants, such as talc or magnesium stearate and, stabilizers. In soft capsules, the active compounds are preferably dissolved or suspended in suitable liquids, such as fatty oils, or liquid paraffin. In addition, stabilizers may be added.

Suitable formulations for parenteral administration include aqueous solutions of the active compounds, e.g., water-soluble salts and alkaline solutions. In addition, suspensions of the active compounds as appropriate oily injection suspensions may be administered. Suitable lipophilic solvents or vehicles include fatty oils, e.g., sesame oil, or synthetic fatty acid esters, e.g., ethyl oleate, triglycerides or polyethylene glycol-400, cremophor, or cyclodextrins. Aqueous injection suspensions may contain substances which increase the viscosity of the suspension include, e.g., sodium carboxymethyl cellulose, sorbitol, or dextran. Optionally, the suspension may also contain suspension stabilizers.

The topical formulations of this disclosure are formulated preferably as oils, creams, lotions, ointments and the like by choice of appropriate carriers. Suitable carriers include vegetable or mineral oils, white petrolatum (white soft paraffin), branched chain fats or oils, animal fats and high molecular weight alcohol (greater than C₁₂). The preferred carriers are those in which the active ingredient is soluble. Emulsifiers, stabilizers, humectants and antioxidants may also be included, as well as agents imparting color or fragrance, if desired. Additionally, transdermal penetration enhancers may be employed in these topical formulations. Examples of such enhancers are found in U.S. Patent Nos. 3,989,816 and 4,444,762.

Creams are preferably formulated from a mixture of mineral oil, self-emulsifying beeswax and water in which mixture of the active ingredient, dissolved in a small amount of an oil, such as almond oil, is admixed. A typical example of such a cream is one which includes approximately 40 parts water, approximately 20 parts beeswax, approximately 40 parts mineral oil and approximately 1 part almond oil.

Ointments may be formulated by mixing a solution of the active ingredient in a vegetable oil, such as almond oil, with warm soft paraffin and allowing the mixture to cool. A typical example of such an ointment is one which includes approximately 30% almond oil and approximately 70% white soft paraffin by weight.

The "effective dose" in the present disclosure relates to the amount of biological or medical responses of active compounds or medical reagents to tissues, systems, animals or individual workers sought by researchers, veterinarians, physicians or other clinicians. The said biological or medical response includes one or more of the following: (1) Prevention of disease: for example, prevention of disease, condition or illness in an individual, who may be vulnerable to the disease, pathology or symptoms, that has not yet been experienced or presented, (2) Suppression: for example, the suppression of an individual who is experiencing or presenting a disease, condition, or symptoms. (i.e., to prevent the further development of the said pathology and / or symptoms), and (3) Improve disease: for example, to improve the disease, condition or disease in the individual who is experiencing or presenting the disease, condition or pathology or symptom of the disease (i.e., reverse the said pathology and/or symptoms). Thus, an unrestricted instance of the "effective dose" composition of the present disclosure may be used to suppress, block or reverse the activation, migration or proliferation of cells, or to effectively treat cancer or improve its symptoms.

The following examples are illustrative, but not limiting, of the method and compositions of the present disclosure. Other suitable modifications and adaptations of the variety of conditions and parameters normally encountered in clinical therapy and which are obvious to those skilled in the art are within the spirit and scope of the invention.

### Example 1

### The prophylactic therapeutic efficacy of hedgehog pathway inhibitor N-(3-(5-isopropyl-1H-imidazol-2-yl)-4-chlorophenyl)-2-trifluoromethyl-4-((3S,5R)-3,4,5-trimethylpiperazin-1-yl)benzamide (compound A) in unilateral pulmonary fibrosis model in SD rats

### Experimental materials:

1. Reagent: Compound A (N-(3-(5-isopropyl-1H-imidazol-2-yl)-4-chlorophenyl)-2-trifluoromethyl-4-((3S,5R)-3,4,5-trimethylpiperazin-1-yl)benzamide, for testing); Nintedanib ethylsulfonate (BIBF1120, positive reference, Shanghai Boyle Chemical Co., Ltd.); Bleomycin hydrochloride for injection (BLM, modeling agent, Nippon Kayaku Co., Ltd.).
2. Vehicle: Normal saline (Anhui Shuanghe Pharmaceutical Co., Ltd.); MC (Sigma); Tween 80 (Aladdin).
3. Preparation of test sample:
   (1) Preparation of modeling agent: preparation of 1 mg/ml solution of BLM: to a bottle of BLM (5 mg), was added 5 ml of saline, then the mixture was shaken gently, set still for 5 min.
   (2) Preparation of vehicle: preparation of vehicle of 0.5% MC+0.2% Tween 80: 100 ml of distilled water was heated to 80 °C, to which add 5 g of methylcellulose, and the mixture was stirred well. To the mixture was add about 400 ml of ice distilled water after removing the heat source, and the mixture was stirred for 30 min, then transferred to a capacity bottle with 1L volume. After restoring to r.t., distilled water was added to the final volume of 1 L, and the mixture was stirring until the solution was clear. 2 ml Tween 80 was added to 1 L solution of 0.5% methyl cellulose, a uniform solution was obtained after vortex, and then the solution was stored at 4°C for use.
   (3) Preparation of solution of BIBF1120: preparation of 6.0 mg/ml solution of BIBF1120: 648 mg of BIBF 1120 was weighed, to which was added 108 ml vehicle of 0.5% of MC and 0.2% Tween 80, and the mixture was vortex mixed uniformly, then the concentration of BIBF 1120 was 6.0 mg/ml. The solution was stored at 4 °C for use for 3 days and then re-formulated.
   (4) Preparation of solution of Compound A:
      preparation of 2.0 mg/ml solution of Compound A: 378 mg of Compound A was weighed, to which was added 189 ml of vehicle of 0.5% of MC and 0.2% Tween 80, and the mixture was vortex mixed uniformly, then the concentration of Compound A is 2 mg/ml. The solution is stored at 4 °C for use for 3 days and then re-formulated.
      preparation of 1.0 mg/ml solution of Compound A: to 81 ml of 2.0 mg/ml solution of Compound A was added 81 ml of vehicle of 0.5% of MC and 0.2% Tween 80, and the mixture was vortex mixed uniformly, then the concentration of Compound A is 1.0 mg/ml. The solution is stored at 4 °C for use for 3 days and then re-formulated.
      preparation of 0.5 mg/ml solution of Compound A: to 54 ml of 1.0 mg/ml solution of Compound A was added 54 ml of vehicle of 0.5% of MC and 0.2% Tween 80, and the mixture was vortex mixed uniformly, then the concentration of Compound A is 0.5 mg/ml. The solution is stored at 4 °C for use for 3 days and then re-formulated.

### Laboratory animals:

40 male SD rats with Grade SPF, weighing 240-260 g, were purchased from Beijing Weitonglihua Experimental Animal Co., Ltd. (animal certificate number: 11400700169069).

### Experimental methods:

40 male SD rats were randomized into Model group (Group 1, n=8), positive drug group BIBF1120 60 mg/kg/d group (Group 2, n=8), Compound A 5 mg/kg/d group (Group 3, n=8), compound A 10 mg/kg/d group (Group 4, n=8) and Compound A 20 mg/kg/d (Group 5, n=8). On the modeling day, rats were anesthetized and exposed to trachea. Bleomycin was injected into trachea of each group (dosage: 3 mg/kg, volume: 1. 0 ml/kg). Oral administration was performed 2 hours after modeling, once a day. On Day 15, all the animals were killed, the left lung was taken, the weight and volume of the left lung were measured, then fixed by formalin, the bronchioles were stained with HE, Masson trichrome. The bronchioles, final bronchiole and small pulmonary artery injury and inflammation invasion were evaluated by semi-quantitative score, and pulmonary fibrosis was evaluated by Ashcraft semi-quantitative score.

**Table 1 Experimental grouping and administration**

| Group | No. of animal | BLM (3.0 mg/kg) | Compound | No. Of left lung pathological examination (D15) | Dosage (mg/kg) | Dose rout | Start time | Stop time |
|---|---|---|---|---|---|---|---|---|
| Group 1 | 8 | √ | - | 8 | 0 | oral | | |
| Group 2 | 8 | √ | BIBF1120 | 8 | 60 | oral | Modeling day | D15 after modeling |
| Group 3 | 8 | √ | Comp A | 8 | 5 | oral | Modeling day | D15 after modeling |
| Group 4 | 8 | √ | Comp A | 8 | 10 | oral | Modeling day | D15 after modeling |
| Group 5 | 8 | √ | Comp A | 8 | 20 | oral | Modeling day | D15 after modeling |

### Experimental results:

### 1. Basic physiological observation of animals during drug administration

During administration, no obvious physiological and behavioral changes were observed in all experimental animals.

### 2. Changes in body weight of all animals during the experiment

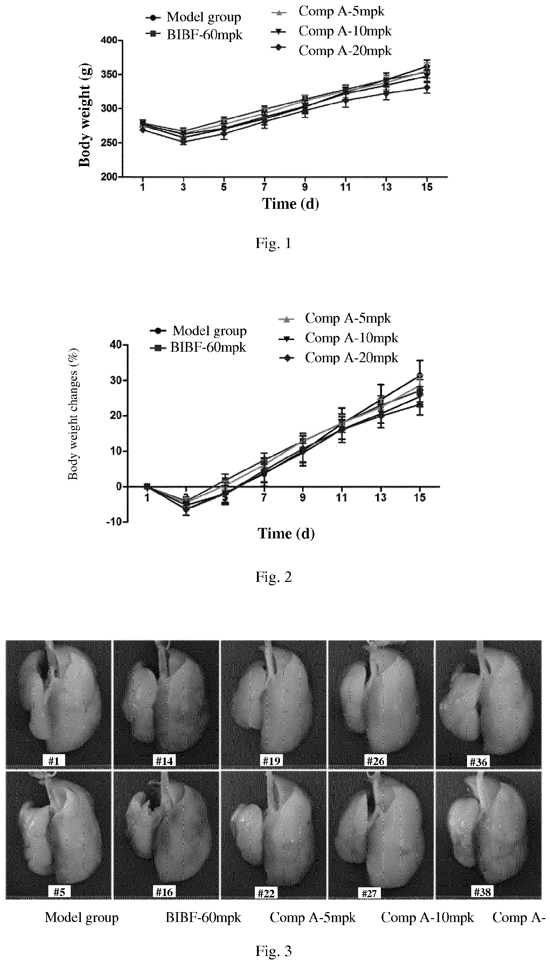

The animals had a slight decrease in body weight for a short period of time (within 6-7 days) during the course of the experiment. The weight of all animals increased gradually as the experiment process (see Fig. 1 and 2 for details).

### 3. Measurement of weight and volume of left lung

The lung volume of each group was reduced after left pulmonary fibrosis for two weeks. The weights of left lung in Compound A-10 mpk and Compound A-20 mpk groups were significantly lower than that in model group (p < 0. 05). At the same time, the weights of left lung in compound A-10 mpk and compound A-20 mpk groups were lower than that in compound A-5 mpk group (p < 0. 05). The weight of left lung in positive control drug BIBF1120 group was lower than that in model group. Compared with model group, the left lung volume of treatment groups showed a decreasing trend, but there was no statistical difference (see Table 2 and Fig. 3-5 for details).

**Table 2 Weight and volume of left lung (mean±sem)**

| Evaluation parameters | Modeling group (n=8) | BIBF1120 60 mg/kg/d (n=8) | Comp A 5 mg/kg/d (n=8) | Comp A 10 mg/kg/d (n=8) | Comp A 20 mg/kg/d (n=8) |
|---|---|---|---|---|---|
| Volume of left lung (cm³) | 1.70 ±0.22 | 1.24±0.17 | 1.38±0.05 | 1.29±0.06 | 1.21±0.09 |
| Weight of left lung (g) | 1.64±0.17 | 1.18±0.14 | 1.37±0.08 | 1.13±0.05 | 1.13±0.08 |

### 4. Detection results of HE staining pathological damage of left lung

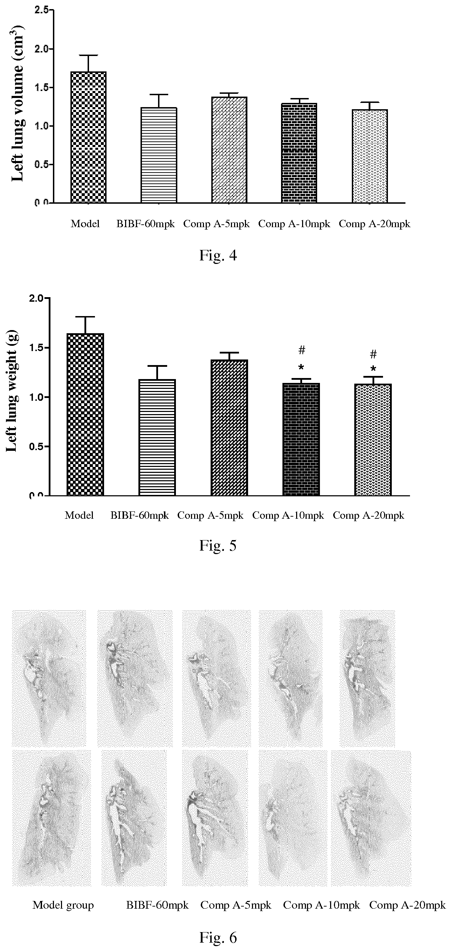

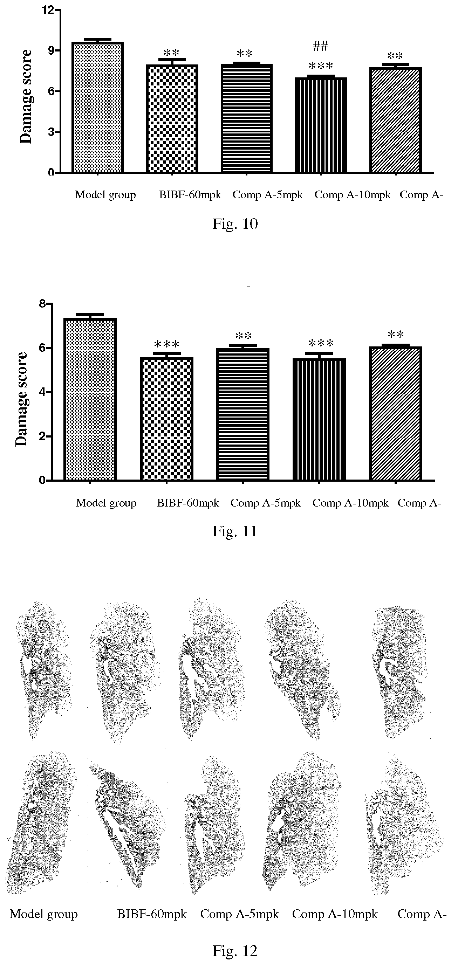

Histological observation of the pathological changes of lung tissue in each group showed obvious lung injury with clear boundary of lung tissue (Fig. 6). Bronchioles, terminal bronchioles, alveolar duct epithelial cells proliferate in varying degrees, some of the epithelium and even the whole layer of epithelium cellularize in goblet, and unequal mucous tissue can be seen in the lumen. There were different degrees of inflammatory cell infiltration in the wall, partial wall thickening, and hyperplasia of smooth muscle and granulation tissue of the outer membrane of tube wall; different degrees of small arterial endothelial cells shedding and proliferation were observed in pulmonary arterioles and arterioles, there were varying degrees of inflammatory cell infiltration in the arterial wall, smooth muscle layer thickening, inflammatory exudation and adventitious granuloma formation (Fig. 7 and 8). The alveolar tissue in the lesion was damaged to different degrees, which was manifested as alveolar epithelium shedding and regeneration, alveolar wall thickening and fibrosis. There were inflammatory exudation of alveolar cavity, inflammatory cell infiltration in alveolar wall and cavity (Fig. 9). According to the lesion area, the terminal bronchioles and the accompanying damage of small pulmonary arteries were divided into fibrosis foci and fibrotic marginal area to evaluate the degree of pathological injury (Table 3). The results showed that the injury of terminal bronchioles and pulmonary arterioles in experimental group treated with Compound A-10 mpk was significantly reduced compared with model group and Compound A-5 mpk group (p<0.001) (Fig. 10), and the analysis of the marginal areas of fibrosis was consistent with the trend of the fibrosis foci (Fig. 11).

**Table 3 Evaluation of injury and inflammatory lesions of left lung (mean±sem)**

| Evaluation parameters | Model group (n=8) | BIBF1120 60 mg/kg/d (n=8) | Comp A 5 mg/kg/d (n=8) | Comp A 10 mg/kg/d (n=8) | Comp A 20 mg/kg/d (n=8) |
|---|---|---|---|---|---|
| Fibrotic lesion edge | 7.28±0.23 | 5.50±0.25 | 5.93±0.20 | 5.45±0.28 | 6.00±0.13 |
| Fibrosis lesion | 9.55±0.31 | 7.88±0.48 | 7.93±0.16 | 6.93±0.21 | 7.68±0.31 |

### 5. Pathological analysis and detection results of Masson trichromatic fibrosis in left lung

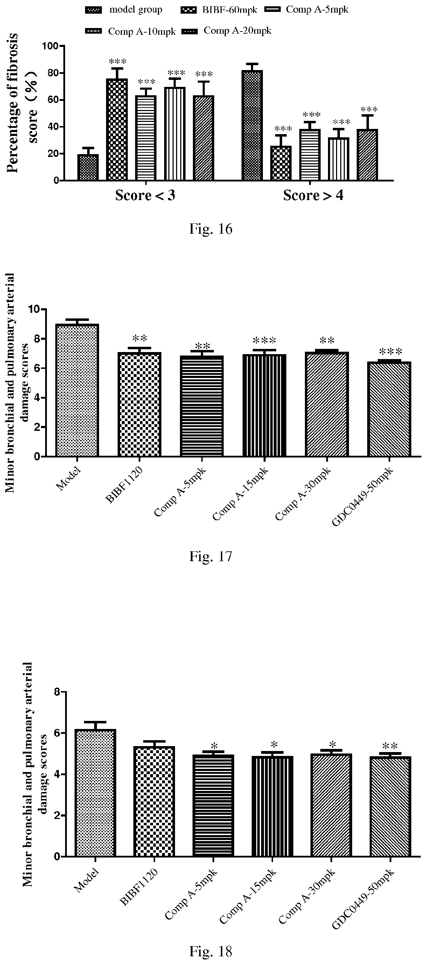

The area of pulmonary fibrosis lesion, the pathological score of pulmonary fibrosis and the grade parameters of fibrosis were evaluated by Masson trichrome staining for pulmonary fibrosis foci in left lung. The results of fibrosis area measurement showed that the area of pulmonary fibrosis lesion induced by BLM was basically uniform among all groups, with no significant difference in lesion area (Fig. 12 and 13, Table 4). The Ashcraft score of pulmonary fibrosis showed that the degree of pulmonary fibrosis in the treatment group of BIBF1120 and Compound A was significantly alleviated compared with that in the model group, with statistically significant differences (p<0.01) (Fig. 14 and 15, Table 4). Although there was no statistically significant difference in the dose-dependent efficacy among the groups treated with compound A, the results showed that the dose-dependent tendency of compound A-lOmpk group was higher than that of compound A-5 mpk group. According to the principle of Ashcraft scoring, in the pulmonary fibrosis scoring, alveolar structure damage (below 3 points) was used as the limit to calculate the cumulative score to evaluate the percentage of pulmonary fibrosis degree. The results showed that more than 80% of the lesions in the model group scored 4 or above. After treatment with BIBF1120 and compound A, there was a significant increase in the lesion score of 3 or less, and more than 60% of the lesion score was below 3 (p<0.001), and a dose-dependent trend was observed in compound A-10 mpk group compared with compound A-5 mpk group at the same time (Fig. 16, Table 4).

**Table 4 Evaluation of left pulmonary fibrosis (mean±sem)**

| Evaluation parameters | Model group (n=8) | BIBF1120 60 mg/kg/d (n=8) | Comp A 5 mg/kg/d (n=8) | Comp A 10 mg/kg/d (n=8) | Comp A 20 mg/kg/d (n=8) |
|---|---|---|---|---|---|
| Fibrosis area | 72.41±7.71 | 77.85±10.90 | 81.64±10.49 | 78.84±4.44 | 76.37±8.33 |
| Fibrosis score | 4.08±0.36 | 3.15±0.65 | 3.33±0.36 | 3.15±0.32 | 3.29±0.63 |
| Fibrosis Score 1-3 (%) | 18.75±15.53 | 75.00±23.90 | 62.50±16.69 | 68.75±19.59 | 71.25±22.95 |
| Fibrosis Score 4-8 (%) | 81.25±15.53 | 25.00±23.90 | 37.50±16.69 | 31.25±19.59 | 28.75±22.95 |

### Conclusion:

Direct tracheal injection of BLM induced significant pulmonary fibrosis in the left lung of rats. The left pulmonary bronchioles, terminal bronchi, and accompanied pulmonary arterioles were significantly damaged after two weeks for modeling. The ashcraft score of pulmonary fibrosis showed that the average score of left pulmonary fibrosis reached 4 points. And the degree of pulmonary fibrosis above 4 points accounted for more than 80%. The positive control drug BIBF1120 and the tested compound A started administration at the same time of tracheal injection of BLM had significant therapeutic effects on inhibiting the progression of BLM-induced pulmonary fibrosis in multiple pathological evaluation indexes; compared with the therapeutic effect of the positive control, BIBF1120, which was administered at 60mg/kg/d, similar inhibitory effects on fibrosis were observed in three doses of compound A (5, 10 and 20 mg/kg). When there was no significant difference in the volume of the fibrotic left lung, compound A (10 and 20 mg/kg) significantly reduced the wet weight of the left lung compared with the model group after treatment, indicating a mild degree of fibrosis in the left lung tissue. Meanwhile, the inhibitory effect of compound A-10 mg/kg on the damage of bronchioles, bronchioles and pulmonary arterioles in fibrotic lesions was significantly better than compound A-5 mg/kg. The ashcraft score of pulmonary fibrosis showed that both the positive drug BIBF1120 and compound A significantly reduced the score of pulmonary fibrosis, and the average score of fibrosis in the entire fibrosis lesion decreased to about 3 points. More significantly, fibrosis lesions below 3 points accounted for more than 60%, and above 4 points was only less than 40%, indicating that the degree of fibrosis in the entire left lung tissue was significantly inhibited. Although the ashcraft score of pulmonary fibrosis showed that there was no statistically significant difference in the dose-effect relationship among the three different dose groups of the tested compound, it was still clearly observed that Compound A-10 mg/kg dose group was superior to Compound A-5 mg/kg dose group, indicating the possibility of the dose-effect relationship.

### Example 2

### The therapeutic efficacy of hedgehog pathway inhibitor N-(3-(5-isopropyl-1H-imidazol-2-yl)-4-chlorophenyl)-2-trifluoromethyl-4-((3S,5R)-3,4,5-trimethylpiperazin-1-yl)benzamide (compound A) in unilateral pulmonary fibrosis model in SD rats

### Experimental materials:

1. Reagent: Compound A (N-(3-(5-isopropyl-1H-imidazol-2-yl)-4-chlorophenyl)-2-trifluoromethyl-4-((3S,5R)-3,4,5-trimethylpiperazin-1-yl)benzamide, for testing); Compound GDC0449 (PharmaBlock Sciences (Nanjing), Inc., for test); Nintedanib ethylsulfonate (BIBF1120, positive control, Shanghai Boyle Chemical Co., Ltd.); Bleomycin hydrochloride for injection (BLM, modeling agent, Nippon Kayaku Co., Ltd.).
2. Vehicle: Normal saline (Anhui Shuanghe Pharmaceutical Co., Ltd.); MC (Sigma); Tween 80 (Aladdin).
3. Preparation of test sample:
   (1) Preparation of modeling agent: preparation of 1 mg/ml solution of BLM: to a bottle of BLM (5 mg), was added 5 ml of saline, then the mixture was shaken gently, set still for 5 min.
   (2) Preparation of vehicle: preparation of vehicle of 0.5% MC+0.2% Tween 80: 100 ml of distilled water was heated to 80 °C, to which add 5 g of methylcellulose, and the mixture was stirred well. To the mixture was add about 400 ml of ice distilled water after removing the heat source, and the mixture was stirred for 30 min, then transferred to a capacity bottle with 1L volume. After restoring to r.t., distilled water was added to the final volume of 1 L, and the mixture was stirring until the solution was clear. 2 ml Tween 80 was added to 1 L solution of 0.5% methyl cellulose, a uniform solution was obtained after vortex, and then the solution was stored at 4°C for use.
   (3) Preparation of solution of BIBF1120: preparation of 6.0 mg/ml solution of BIBF1120: 648 mg of BIBF 1120 was weighed, to which was added 108 ml vehicle of 0.5% of MC and 0.2% Tween 80, and the mixture was vortex mixed uniformly, then the concentration of BIBF 1120 was 6.0 mg/ml. The solution was stored at 4 °C for use for 3 days and then re-formulated.
   (4) Preparation of solution of Compound A:
      preparation of 2.0 mg/ml solution of Compound A: 378 mg of Compound A was weighed, to which was added 189 ml of vehicle of 0.5% of MC and 0.2% Tween 80, and the mixture was vortex mixed uniformly, then the concentration of Compound A was 2 mg/ml. The solution was stored at 4 °C for use for 3 days and then re-formulated.
      preparation of 1.0 mg/ml solution of Compound A: to 81 ml of 2.0 mg/ml solution of Compound A was added 81 ml of vehicle of 0.5% of MC and 0.2% Tween 80, and the mixture was vortex mixed uniformly, then the concentration of Compound A was 1.0 mg/ml. The solution was stored at 4 °C for use for 3 days and then re-formulated.
      preparation of 0.5 mg/ml solution of Compound A: to 54 ml of 1.0 mg/ml solution of Compound A was added 54 ml of vehicle of 0.5% of MC and 0.2% Tween 80, and the mixture was vortex mixed uniformly, then the concentration of Compound A was 0.5 mg/ml. The solution was stored at 4 °C for use for 3 days and then re-formulated.
   (5) Preparation of solution of Compound GDC0449:
      preparation of 5.0 mg/ml solution of Compound GDC0449: 384 mg of Compound GDC0449 was weighed, to which was added 5 ml of vehicle of 0.5% of MC and 0.2% Tween 80, and the mixture was vortex mixed uniformly, and 71.8 ml of vehicle of 0.5% of MC and 0.2% Tween 80 was added again, then the concentration of Compound GDC0449 was 5.0 mg/ml. The solution was stored at 4 °C for use for 3 days and then re-formulated.

### Laboratory animals:

48 male SD rats with Grade SPF, weighing 240-260 g, were purchased from Beijing Weitonglihua Experimental Animal Co., Ltd. (animal certificate number: 11400700188276).

### Experimental methods:

48 male SD rats were randomly divided into Model group (Group 1, 10 ml/kg of vehicle, n=8), positive control drug BIBF1120 100 mg/kg/d group (Group 2, n=8), Compound A 5 mg/kg/d group (Group 3, n=8), compound A 15 mg/kg/d group (Group 4, n=8), Compound A 30 mg/kg/d (Group 5, n=8), and Compound GDC0449 50 mg/kg/d (Group 6, n=8). On the modeling day, rats were anesthetized and exposed to trachea. Bleomycin was injected into left trachea of each group (dosage: 3 mg/kg, volume: 1.0 ml/kg). Oral administration was performed on Day 8 after modeling, once a day. On Day 22, according to the KCI animal euthanasia SOP, animals in each group were given an overdose of sodium barbiturate and sacrificed under intraperitoneal anesthesia, the left lung was taken, the weight and volume of the left lung were measured, then fixed by formalin, the bronchioles were stained with HE, Masson trichrome. The bronchioles, final bronchiole and small pulmonary artery injury and inflammation invasion were evaluated by semi-quantitative score, and pulmonary fibrosis was evaluated by Ashcraft semi-quantitative score. Immunohistochemical staining of α-SMA, Type I collagen (Collagen-I) were performed at the same time, a full scan was performed by Hamamatsu NanoZoomer Digital Pathology (S210), the α-SMA positive area was semiquantitatively calculated from the unit area of the whole sheelt in pulmonary fibrosis foci, and the positive staining area of type-I collagent per unit of area was calculated.

**Table 5 Experimental grouping and administration**

| Group | No, of animals | BLM (3.0 mg/kg) | Compound | Dosage (mg/kg) | Dose rout | Start time | Terminal Time |
|---|---|---|---|---|---|---|---|
| Group 1 | 8 | √ | - | 0 | oral | | |
| Group 2 | 8 | √ | BIBF1120 | 100 | oral | D8 of modeling | D22 after modeling |
| Group 3 | 8 | √ | Comp A | 5 | oral | D8 of modeling | D22 after modeling |
| Group 4 | 8 | √ | Comp A | 15 | oral | D8 of modeling | D22 after modeling |
| Group 5 | 8 | √ | Comp A | 30 | oral | D8 of modeling | D22 after modeling |
| Group 6 | 8 | √ | GDC0449 | 50 | oral | D8 of modeling | D22 after modeling |

### Experiment results:

### 1. Basic physiological observation of animals during drug administration

During administration, no obvious physiological and behavioral changes were observed in all experimental animals. No obvious pathological changes were found in the left lung of some animals at the end of dissection, and no obvious pathological changes of pulmonary fibrosis were confirmed by lung histopathology. Accordingly, these animals were identified as unsuccessful models and excluded from the final statistics (Table 6).

**Table 6 Animals entering the group at the end**

| Group | No. of animals for modeling | Compound therapy | No. of left lung pathological examination (D22) |
|---|---|---|---|
| Group 1 | 8 | Vehcile | 5(3 animals for modeling failed) |
| Group 2 | 8 | BIBF1120 | 6(2 animals for modeling failed) |
| Group 3 | 8 | Comp A | 5(3 animals for modeling failed) |
| Group 4 | 8 | Comp A | 7(1 animal for modeling failed) |
| Group 5 | 8 | Comp A | 7(1 animal for modeling failed) |
| Group 6 | 8 | GDC0449 | 8 |

### 2. Changes in body weight of all animals during the experiment

The animals had a slight decrease in body weight for a short period of time (within 6-7 days) during the course of the experiment. The weight of all animals increased gradually as the experiment process. There was no significant difference in average body weight among the experimental groups.

### 3. Measurement of weight and volume of left lung

The lung volume of each group was reduced after left pulmonary fibrosis for three weeks. There was no significant difference between the groups (p>0.05). At the same time, there was no significant difference in left lung weight between the groups after perfusion (Table 7).

**Table 7 Weight and volume of left lung mean±sem)**

| Evaluation parameter | Model group | BIBF1120 100 mpk | Comp A-5 mpk | Comp A-15 mpk | Comp A-30 mpk | GDC0449-50 mpk |
|---|---|---|---|---|---|---|
| Volume of left lung (cm³) | 1.04±0.04 | 1.23±0.09 | 1.34±0.14 | 1.41±0.21 | 1.40±0.09 | 1.28±0.10 |
| Weight of left lung (g) | 1.13±0.06 | 1.31±0.09 | 1.60±0.33 | 1.40±0.21 | 1.44±0.14 | 1.29±0.09 |

### 4. Detection results of HE staining pathological damage of left lung

Histological observation of the diseased lung tissue in each group showed clear lung tissue boundaries and significant lung injury. There were different degrees of proliferation of bronchioles, terminal bronchioles and alveolar duct epithelial cells in the lesion, goblet cellularization in part of the epithelium and even the whole layer of the epithelium, and different amounts of mucus tissue in the lumen. Inflammatory cell infiltration, partial tube wall thickening, smooth muscle hyperplasia and granulation tissue hyperplasia of the outer membrane of tube wall were observed in different degrees; here were different degrees of small arterial endothelial cells falling off and proliferating in the pulmonary arterioles and arterioles, inflammatory cells infiltrating to different degrees in the arterial wall, thickened smooth muscle layer, inflammatory exudation and the formation of adventitious granuloma. The alveolar tissue in the lesion was damaged to different degrees, which was manifested as alveolar epithelium shedding and regeneration, alveolar wall thickening and fibrosis. There were inflammatory exudation of alveolar cavity, inflammatory cell infiltration in alveolar wall and cavity. According to the lesion area, the terminal bronchioles and the accompanying damage of small pulmonary arteries were divided into fibrosis foci and fibrotic marginal area to evaluate the degree of pathological injury (Table 8). The results showed that it significantly reduced the damage and inflammatory response of terminal bronchioles within fibrosis lesions and pulmonary small artery, treated with positive control BIBF1120, Compound A and GDC0449 for 14 consecutive days, with significant difference compared with model group (p < 0.001) (Figure 17), and the analysis results of fibrosis in edge area were consistent with trends within the focal fibrosis (Fig 18). There were no significant dose-dependent changes among the three dose groups, i.e., low, medium, high dose groups for the test compound A.

**Table 8 Evaluation of injury and inflammatory lesions of left lung (mean±sem)**

| Evaluation parameter | Model group | BIBF1120 100 mpk | Comp A-5 mpk | Comp A-15 mpk | Comp A-30 mpk | GDC0449-50 mpk |
|---|---|---|---|---|---|---|
| Fibrotic lesion edge | 6.15±0.38 | 5.31±0.28 | 4.90±0.15 | 4.82±0.24 | 4.95±0.20 | 4.80±0.22 |
| Fibrosis lesion | 8.92±0.38 | 7.00±0.36 | 6.75±0.40 | 6.89±0.34 | 7.03±0.18 | 6.38±0.17 |

### 5. Pathological analysis and detection results of Masson trichromatic fibrosis in left lung

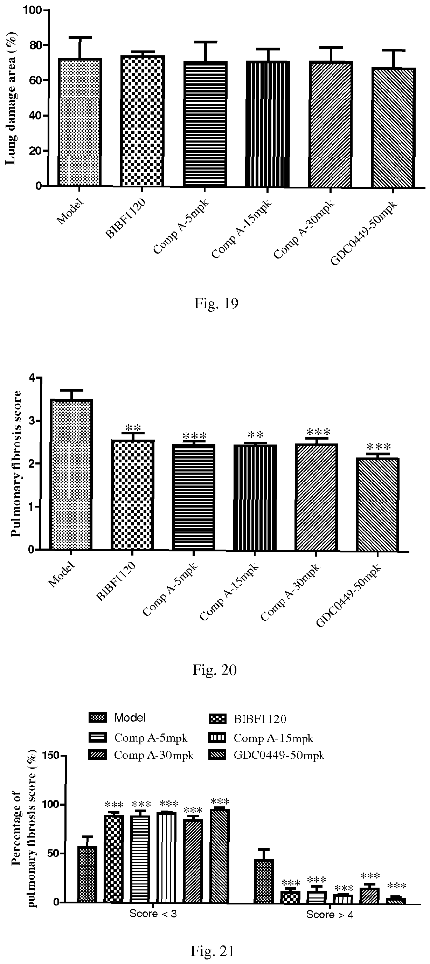

The area of pulmonary fibrosis lesion, the pathological score of pulmonary fibrosis and the grade parameters of fibrosis were evaluated by Masson trichrome staining for pulmonary fibrosis foci in left lung. The results of fibrosis area measurement showed that the area of pulmonary fibrosis lesion induced by BLM was basically uniform among all groups, with no significant difference in lesion area (Fig. 19, Table 9). The destruction of alveolar structure, the fibrosis of alveolar wall and the fibrosis mass in alveolar cavity can be clearly seen by Masson's trichrome staining. Pulmonary fibrosis was significantly relieved after treatment with the compound. The Ashcraft score of pulmonary fibrosis showed that the degree of pulmonary fibrosis treated with BIBF1120, Compound A and GDC0449 was significantly better than that in the model group, with significant difference by statistic analysis (p < 0.01) (Fig 20, Table 9). There was no significant statistic difference in dose-dependent effect between each dose group of the test compound A. According to the principle of Ashcraft scoring, in the pulmonary fibrosis scoring, alveolar structure damage (below 3 points) was used as the limit to calculate the cumulative score to evaluate the percentage of pulmonary fibrosis degree. The results showed that more than 60% of the lesions in the model group had a score of 4 or more. After the treatment of BIBF1120, compound A and GDC0449, the lesion area with score of 3 or below increased significantly, and the area above 80% was below 3 (p < 0.001) (Fig. 21, Table 9).

**Table 9 Evaluation of fibrosis in left pulmonary (mean±sem)**

| Evaluation parameters | Model group | BIBF1120 100 mpk | Comp A-5 mpk | Comp A-15 mpk | Comp A-30 mpk | GDC0449-50 mpk |
|---|---|---|---|---|---|---|
| Fibrosis area | 72.09±5.61 | 73.81±1.14 | 70.61±5.23 | 71.16±2.81 | 71.27±3.21 | 67.96±3.70 |
| Fibrosis score | 3.48±0.23 | 2.55±0.18 | 2.44±0.11 | 2.44±0.07 | 2.47±0.16 | 2.16±0.11 |
| Fibrosis Score 1-3 (%) | 56.00±11.23 | 88.33±4.01 | 88.00±5.83 | 91.43±1.43 | 84.29±4.81 | 95.00±2.67 |
| Fibrosis Score 4-8 (%) | 44.00±11.23 | 11.67±4.01 | 12.00±5.83 | 8.57±1.43 | 15.71±4.81 | 5.00±2.67 |

### 6. Immunohistochemical results of left lung tissue

### (1) Immunohistochemical results of Type I collagen

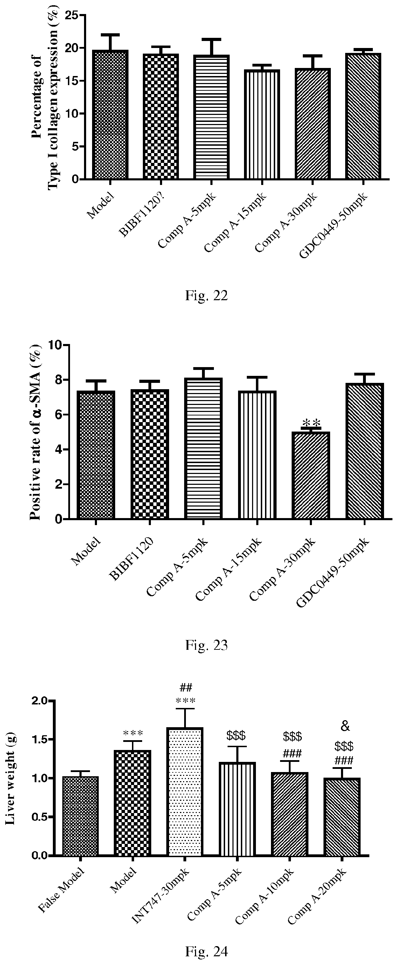

Immunohistochemical results of Type I collagen showed a very small amount of Type I collagen tissue in the alveolar wall of sham operation group. Diffuse Type I collagen deposition in fibrosis tissue, with fine reticular distribution, and some of them showed collagen-like structure were observed in the lesions of pulmonary fibrosis in each group. Type I collagen deposition with uneven density and distribution can be seen in the alveolar walls with alveolar structure remaining in the lesion, resulting in thickening and destruction of the remaining alveolar walls due to collagen deposition. The results of semi-quantitative immunohistochemical analysis of Type I collagen were shown in Fig. 22: there was no significant difference in the amount of collagen deposition in the fibrotic lesions of model group compared with that of the positive drug BIBF1120, Compound A (5 mpk) and GDC0449 groups. The percentage of Type-I collagen protein decreased at the dose of 30 mg/kg/d and 15 mg/kg/d for the tested Compound A, but there was no statistical significance (Table 10).

**Table 10 Expression percentage of Type I collagen mean±sem)**

| Evaluation parameters | Model group (n=4) | BIBF1120 100 mpk (n=6) | Comp A-5 mpk (n=5) | Comp A-15 mpk (n=7) | Comp A-30 mpk (n=7) | GDC0449-50 mpk (n=8) |
|---|---|---|---|---|---|---|
| Expression of Type I collagen (%) | 19.54±2.48 | 18.99±1.19 | 18.76±2.54 | 16.48±0.93 | 16.77±2.03 | 19.06±0.70 |

### (2) Immunohistochemical results of α-SMA

The immunohistochemical results of α-SMA showed diffuse proliferation of myofibroblasts (α-SMA positive cells) in the foci of pulmonary fibrosis, spreading over the fibrosis tissue, the alveolar wall of the lesion, and the bronchial adventitia and the non-arteriole adventitia of the lesion. The results of semi-quantitative analysis of α-SMA positive staining showed that there was no significant difference in positive rate of α-SMA in fibrosis lesions per unit area in model group compared with the positive drugs BIBF1120 and GDC0449. The percentage of α-SMA protein significantly decreased at the dose of 30 mg/kg/d of the tested compound A compared with the model group, and there was a significant dose dependence of Compound A (Fig. 23, Table 11).

**Table 11 Percentage of α-SMA protein expression mean±sem**

| Evaluation parameters | Model group (n=4) | BIBF1120 100 mpk (n=6) | Comp A-5 mpk (n=5) | Comp A-15 mpk (n=7) | Comp A-30 mpk (n=7) | GDC0449-50 mpk (n=8) |
|---|---|---|---|---|---|---|
| Expression of α-SMA protein (%) | 7.29±0.64 | 7.36±0.55 | 8.06±0.59 | 7.30±0.84 | 4.96±0.26** | 7.74±0.59 |

### Conclusion:

Direct tracheal injection of BLM induced significant pulmonary fibrosis in the left lung of rats. The left pulmonary bronchioles, terminal bronchi, and accompanied pulmonary arterioles were damaged, and alveolus pulmonis were significantly damaged after three weeks for modeling. The ashcraft score of pulmonary fibrosis showed that the average score of left pulmonary fibrosis reached 4 points. And the degree of pulmonary fibrosis above 4 points accounted for more than 60%. The treatment for 14 consecutive days of the positive drug BIBF1120, the tested Compound A and GDC0449 started dose on Day 8 after modeling had significant therapeutic effects on inhibiting the progression of BLM-induced pulmonary fibrosis in multiple pathological evaluation indexes. Compared with the therapeutic effect of the positive control, BIBF1120 at the dose of 100mg/kg/d, similar inhibitory effects on fibrosis were observed in three doses of compound A (5, 15 and 30 mg/kg). The treatment effect of GDC0449 at 50 mg/kg/d for 14 consecutive days was the same as that of BIBF1120. The ashcraft score of pulmonary fibrosis showed that both the positive drugs and the tested compounds significantly reduced the score of pulmonary fibrosis, and the average score of the entire fibrosis lesion was reduced to about 3 points. More significantly, the degree of fibrosis below 3 points accounted for more than 80%, and that above 4 points was less than 20%, indicating that the degree of fibrosis in the entire left lung tissue was significantly inhibited. The ashcraft score of pulmonary fibrosis showed that no significant dose-effect relationship was observed in the therapeutic efficacy test of the three different dose groups of the tested compound A, but there was a dose-dependent change in the protein expression of α-SMA at three doses of Compound A, and the protein expression was significantly reduced at the dose of 30 mg/kg/d. After 14 days of treatment, there was no significant change in the expression of Type I collagen in the lung tissue of compound A group compared with model group. There was no significant regulation of α-SMA and type I collagen expression for the positive drug BIBF1120 and the tested compound GDC0449 at 50 mg/kg/d for 14 consecutive days.

According to the analysis of experimental results of compounds A and GDC0449 in this study, both compounds have a certain effect on inhibiting pulmonary fibrosis, which is mainly manifested in the improvement of pulmonary pathological changes. A dose-dependent change in the protein expression of α-SMA at three doses of Compound A, and the protein expression was significantly reduced at 30mg/kg/d, however, there was no clear regulation of Type I collagen. There was no regulation of α-SMA and type I collagen in GDC0449 group.

### Example 3

### The prophylactic therapeutic efficacy study of hedgehog pathway inhibitor N-(3-(5-isopropyl-1H-imidazol-2-yl)-4-chlorophenyl)-2-trifluoromethyl-4-((3S,SR)-3,4,5-trimethylpiperazin-1-yl)benzamide (compound A) in liver fibrosis model in C57BL/6 mice induced by CCl₄

### Experimental materials:

1. Reagent: Compound A (N-(3-(5-isopropyl-1H-imidazol-2-yl)-4-chlorophenyl)-2-trifluoromethyl-4-((3S,5R)-3,4,5-trimethylpiperazin-1-yl)benzamide, for testing); Obercholic acid (INT747, positive control substance, Nanjing KCI Biotechnology Co., Ltd.); CCl₄ (modeling agent, Chinese Medicine Group Chemical Reagent Co., Ltd.); olive Oil (modeling agent, Italy Orney Co., Ltd.).
2. Vehicle: methylcellulose (MC, Sigma); Tween 80 (Aladdin).
3. Preparation of test sample:
   (1) Preparation of modeling agent: CCl₄ was dissolved in olive oil at a dose of 0.5 µl/g, and a solution of CCl₄: olive oil = 1:4 was prepared.
   (2) Preparation of vehicle: preparation of vehicle of 0.5% MC+0.2% Tween 80: 100 ml of distilled water was heated to 80 °C, to which was added 5 g of methylcellulose, and the mixture was stirred well. To the mixture was add about 400 ml of ice distilled water after removing the heat source, and the mixture was stirred for 30 min, then transferred to a capacity bottle with 1L volume. After restoring to r.t., distilled water was added to the final volume of 1 L, and the mixture was stirring until the solution was clear. 2 ml Tween 80 was added to 1 L solution of 0.5% methyl cellulose, a uniform solution was obtained after vortex, and then the solution was stored at 4°C for use.
   (3) Preparation of solution of Obercholic acid:
      preparation of 3 mg/ml solution of Obercholic acid: 30 mg of Obercholic acid was weighed, to which was added 5 ml vehicle to grind, and another 5 ml vehicle was added after Obercholic acid was completely refined, and the mixture was vortex mixed uniformly, then the concentration of Obercholic acid was 3 mg/ ml. The solution was stored at 4 °C for use for 3 days and then re-formulated.
   (4) Preparation of solution of Compound A:
      preparation of 2.0 mg/ml solution of Compound A: 378 mg of Compound A was weighed, to which was added 189 ml of vehicle of 0.5% of MC and 0.2% Tween 80, and the mixture was vortex mixed uniformly, then the concentration of Compound A is 2 mg/ml. The solution was stored at 4 °C for use for 3 days and then re-formulated.
      preparation of 1.0 mg/ml solution of Compound A: to 81 ml of 2.0 mg/ml solution of Compound A was added 81 ml of vehicle of 0.5% of MC and 0.2% Tween 80, and the mixture was vortex mixed uniformly, then the concentration of Compound A was 1.0 mg/ml, The solution was stored at 4 °C for use for 3 days and then re-formulated.
      preparation of 0.5 mg/ml solution of Compound A: to 54 ml of 1.0 mg/ml solution of Compound A was added 54 ml of vehicle of 0.5% of MC and 0.2% Tween 80, and the mixture was vortex mixed uniformly, then the concentration of Compound A was 0.5 mg/ml, The solution was stored at 4 °C for use for 3 days and then re-formulated.

### Laboratory animals:

56 male C57BL/6 mice with Grade SPF, weighing 25-27 g, were purchased from Shanghai Lingchang Biotechnology Co., Ltd. (animal certificate number: 2013001821552).

### Experimental methods:

56 male C57BL/6 mice were randomize divided into false Model group (Group 1, n=6), Model group (Group 2, n=10), positive drug Obercholic acid group (Group 3, n=10, 30 mg/kg/d), compound A low-dose group (Group 4, n=10, 5 mg/kg/d), Compound A medium-dose (Group 5, n=10, 10 mg/kg/d), and Compound A high-dose group (Group 6, n=10, 20 mg/kg/d). Oral molding agent for molding three times a week, for 4 weeks, weighing before each dose. Compound A was administered orally once a day for a total of 28 days from the day of modeling. The model group was given an equal volume of drug vehicle and weighed before each dose. All animals fasted for 6 hours from 24 hours after the end of the last dose. Serum was collected and the concentrations of AST, ALT and TBIL in serum were detected. All animals were euthanized, the livers were collected, weighed, and photos of the gross anatomy of the livers were taken. The liver tissue was fixed in 10% formalin solution for histopathological examination, H&E staining and Sirius red staining.

**Table 12 Experimental grouping and administration**

| Group | No of animals | Compound | Concentration of drug | Dose rout | Dosage | Dose volume | Dose frequency |
|---|---|---|---|---|---|---|---|
| Group 1 | 6 | Vehicle | - | oral | - | 10 ml/kg | qd |
| Group 2 | 10 | Vehicle | - | oral | - | 10 ml/kg | qd |
| Group 3 | 10 | Obercholic acid | 3 mg/ml | oral | 30 mg/kg | 10 ml/kg | qd |
| Group 4 | 10 | Comp A | 0.5 mg/ml | oral | 5 mg/kg | 10 ml/kg | qd |
| Group 5 | 10 | Comp A | 1 mg/ml | oral | 10 mg/kg | 10 ml/kg | qd |
| Group 6 | 10 | Comp A | 2 mg/ml | oral | 20 mg/kg | 10 ml/kg | qd |

### Experiment results:

### 1. Basic physiological observation of animals during drug administration

During the course of the experiment, there were no changes in abnormal behavior, food intake and excreta, including faeces and urine.

### 2. Changes in body weight of all animals during the experiment

After oral administration of CCl₄ for 24 hours, the animals lost weight slightly, then recovered slowly, and recovered before the next oral administration of CCl₄, showing a "volatility" change. In the high-dose group (20 mg/ kg) of Compound A, the body weight increased slowly after a week, and no significant body weight growth was started after two weeks. There was significant difference at the same time point from the other groups.

### 3. Changes of ratio of liver weight and liver body

In the model group, the surface of the liver was rough, and dull, and fine granular changes were observed. The surface of Obercholic acid treated group was rough and the change of dispersive fine particles was significantly less than that of model group. The changes of liver surface in compound A treatment group were slightly alleviated compared with the model group. After 4 weeks of the experiment, the liver of the model group and the positive drug Obercholic acid group increased significantly. There was no significant increase in liver in the treatment group of Compound A compared with the false model group. The change of liver weight/body weight ratio was consistent with the change of liver weight (Fig 24-25).

### 4. The results of biochemical detection in peripheral blood serum

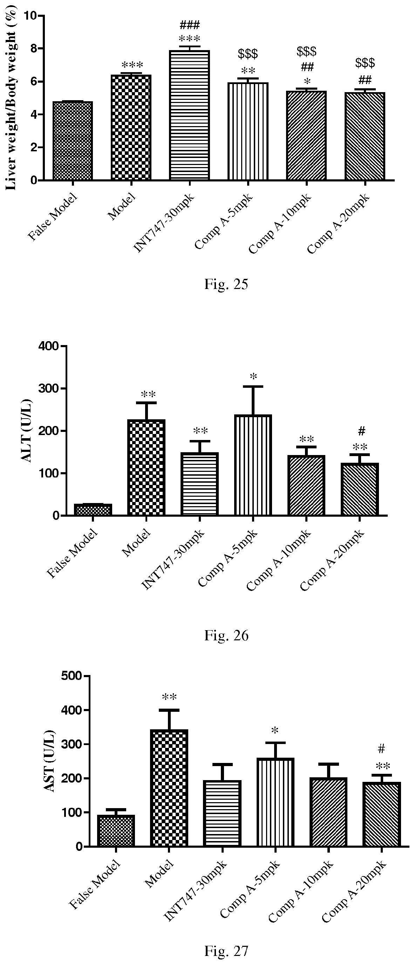

The level of ALT, AST, TBIL in animal peripheral blood of model group was significantly increased after oral administration of CCl₄ for 4 weeks. Compared with the model group, the level of ALT, AST, TBIL in peripheral blood of the Obercholic acid group decreased, but there was no statistical significance. After four weeks of treatment of Compound A, there was a decreasing trend of dose-dependent ALT and AST, of which the high dose group of compound A was significantly lower than that of the model group (p < 0.05), but TBIL was not significantly decreased (Fig. 26-28).

### 5. Histopathological results

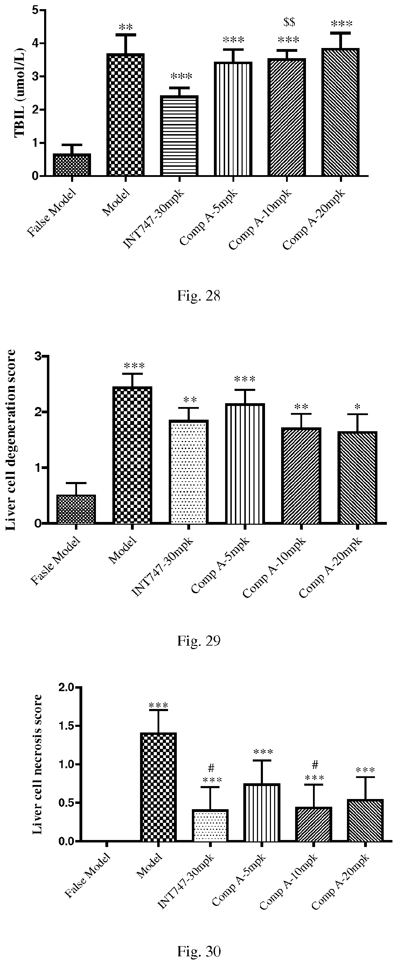

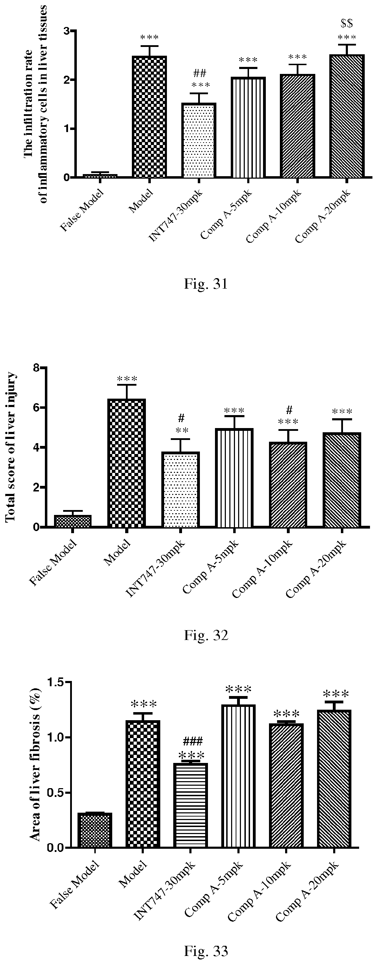

Histological observation with HE staining revealed diffuse lamellar hepatocyte hydrogenicity induced by CCl₄ to varying degrees; multiple, multifocal, portal - centered hepatocyte necrosis. Multiple focal, flaky inflammatory cell infiltration, distributed around the portal area, central vein peripheral and necrotic hepatocyte area. Hepatocyte division and hepatocyte regeneration can be seen in some areas. The different degree of hepatocellular degeneration, necrosis and the reduction of inflammatory cell infiltration were observed after treatment with compound A in each dose group. The results of liver tissue injury score showed that obercholic acid significantly decreased hepatocyte necrosis and inflammatory cell infiltration. The total score of liver cell injury was significantly different from that of the model group. The treatment groups of Compound A had a tendency to reduce hepatocellular degeneration, necrosis and inflammatory cell infiltration, but the overall score of hepatocyte injury was not significantly different from that of the model group (Fig. 29-32). This Sirius red stain demonstrated uneven collagen deposition in the liver tissue. Fibrosis was mostly distributed in the central venous area, portal area and between the hepatic lobules, no clear bridging fibrosis and pseudolobule tissue like. The results of quantitative analysis of the whole scanning liver fibrosis area with Sirius scarlet staining showed that the fibrosis was significantly reduced in the treatment group with obercholic acid, which was significantly different from that in the model group. There was no significant difference in hepatic fibrosis area between each treatment group of compound A and model group (Fig33).

### Conclusion:

Oral administration of CCl₄ three times a week for four weeks successfully induced chronic liver fibrosis model. Around the model, significant liver tissue damage and diffuse hepatic fibrosis were observed. The histopathological score of liver injury was scored 6 on average, the area of hepatic fibrosis reached 1.2% (the inherent fibrosis area of normal liver tissue was 0.3%), and the corresponding serum ALT, AST and TBIL were increased. The efficacy response trend of serum enzyme, liver histology and other parameters could be observed for the positive tested compound Obercholic acid at 30 mg/kg daily for four consecutive weeks, but there was no statistic difference. Slight reductions in serum ALT, AST, and TBIL were observed at different doses (5 mg/kg, 10 mg/kg, and 20 mg/kg) of tested compound A daily for four consecutive weeks, and there were statistically significant differences in ALT and AST between the high-dose compound A group and the model group. The analysis results of liver histopathological showed that the liver injury score in the three dose groups decreased from 6 points in the model group to 4-5 points. The main manifestations were inhibition of hepatocyte degeneration and necrosis. However, it had little effect on the inflammatory response of liver tissue. The area of liver tissue fibrosis decreased from 1.2% to 0.8% in the model group, indicating a trend of inhibition of fibrosis, but there was no statistical difference. At the same time, there was no significant dose-dependent change among the three dose groups of the tested compound A.

According to the analysis of the experimental results of three different doses of compound A tested in this study, compound A has a certain therapeutic trend of inhibiting CCl₄-induced liver fibrosis, which was mainly manifested in reducing the degree of liver cell degeneration and necrosis as well as fibrosis, and there was no clear therapeutic effect of inhibiting inflammatory reaction.

### Example 4

### The therapeutic efficacy study of hedgehog pathway inhibitor N-(3-(5-isopropyl-1H-imidazol-2-yl)-4-chlorophenyl)-2-trifluoromethyl-4-((3S,5R)-3,4,5-trimethylpiperazin-1-yl)benzamide (compound A) in lung fibrosis model in mice induced by Bleomycin

### Experimental materials:

1. Reagent: Compound A (N-(3-(5-isopropyl-1H-imidazol-2-yl)-4-chlorophenyl)-2-trifluoromethyl-4-((3S,5R)-3,4,5-trimethylpiperazin-1-yl)benzamide, for testing); Nintedanib (BIBF1120, positive reference, DC Chemical); Bleomycin (BLM, modeling agent, MedChem Express).
2. Vehicle: 0.9% injection of Sodium Chloride (Chenxin Pharmaceutical Co., Ltd.); methylcellulose (MC, Sigma); Tween 80 (Aladdin).
3. Preparation of test sample:
   (1) Preparation of modeling agent: preparation of 0.33 mg/ml solution of BLM: in aseptic environment, 1.65mg of BLM was weighed, and dissolved in 5ml of 0.9% injection of sodium chloride by vortex, placed in 10 ml brown bottle, and kept in ice box at 4 °C.
   (2) Preparation of vehicle: preparation of vehicle of 0.5% MC+0.2% Tween 80 (MCT): to 1000 ml of fresh prepared 0.5% methylcellulose solution was add 2 ml of Tween 80, and then the solution was stored at 4°C.
   (3) Preparation of solution of BIBF1120: preparation of 5.0 mg/ml solution of BIBF1120: 50.00 mg of BIBF 1120 was weighed, and dissolved in 10 ml of MCT by vortex, which was now used and stored without light.
   (4) Preparation of solution of Compound A:
      preparation of 1.0 mg/ml solution of Compound A: 4.50 mg of Compound A was weighed, and dissolved in 4.50 ml MCT by ultrasonic crushing and vortex. It was now used and stored without light.
      preparation of 2.0 mg/ml solution of Compound A: 18.0 mg of Compound A was weighed, and dissolved in 9.00 ml MCT by ultrasonic crushing and vortex. It was now used and stored without light.

### Laboratory animals:

60 male C57BL/6 mice, 8 weeks old, weighing about 25 g, were purchased from Beijing China Fukang Bio-tech Co., Ltd. (animal certificate number: 11401300053752).

### Experimental methods:

60 male C57BL/6 mice were randomize into 6 groups: Blank control group (Group 1, n=10), model group (Group 2, BLM, n=10), Compound A 10 mpk group (Group 3, n=10), compound A 20 mpk group (Group 4, n=10), Compound A 20mpk + BIBF1120 50mpk (Group 5, n=10), and BIBF1120 50mpk positive reference group (Group 6, n=10). One week after the mice were acclimated to the environment, 10 mice in the blank control group were given 50 µl of normal saline in the trachea, and other 50 mice were given 50 µl BLM (0.66mg/kg, 2ml/kg) in the trachea. After BLM infusion, 50 mice in BLM infusion group were randomly assigned to start treatment on the 5th day, once a day, with a treatment cycle of 20 days. The whole process was 25 days. After the experiment, the lung tissues were fixed in 10% neutral formalin, dehydrated by alcohol gradient, embedded with paraffin, stained by hematoxylin-eosin (H&E) and Masson trichrome staining. And the inflammatory area and degree of fibrosis of the lung tissues in mice were evaluated by APERIO ScanScope. Immunohistochemical staining of lung tissue was performed to quantify α-SMA positive fibroblasts in lung tissue and to calculate the percentage (%) of α-SMA staining in the entire lung stroma.

**Table 13 Experimental grouping and administration**

| Group | No. of animals | BLM (0.66mg/kg) | Compound | Dosage (mg/kg) | Dose route | Start time | Terminal time |
|---|---|---|---|---|---|---|---|
| Group 1 | 10 | × | Vehicle | - | oral | D5 of modeling | D25 after modeling |
| Group 2 | 10 | √ | Vehicle | - | oral | D5 of modeling | D25 after modeling |
| Group 3 | 10 | √ | Comp A | 10 | oral | D5 of modeling | D25 after modeling |
| Group 4 | 10 | √ | Comp A | 20 | oral | D5 of modeling | D25 after modeling |
| Group 5 | 10 | √ | Comp A+ BIBF1120 | A: 20 BIBF1120: 50 | oral | D5 of modeling | D25 after modeling |
| Group 6 | 10 | √ | BIBF1120 | 50 | oral | D5 of modeling | D25 after modeling |

### Experimental results:

### 1. Basic physiological observation of animals during drug administration

During administration, the mice had normal intake and drinking water, no animal physiology, abnormal behavior and death.

### 2. Changes in body weight of all animals during the trial

After BLM induction, the body weight of the model group was significantly lower than that of the control group (p < 0.05); after compound treatment, there was no significant body weight change and no statistic difference between each group.

### 3. Results of collagen content in bronchoalveolar lavage fluid

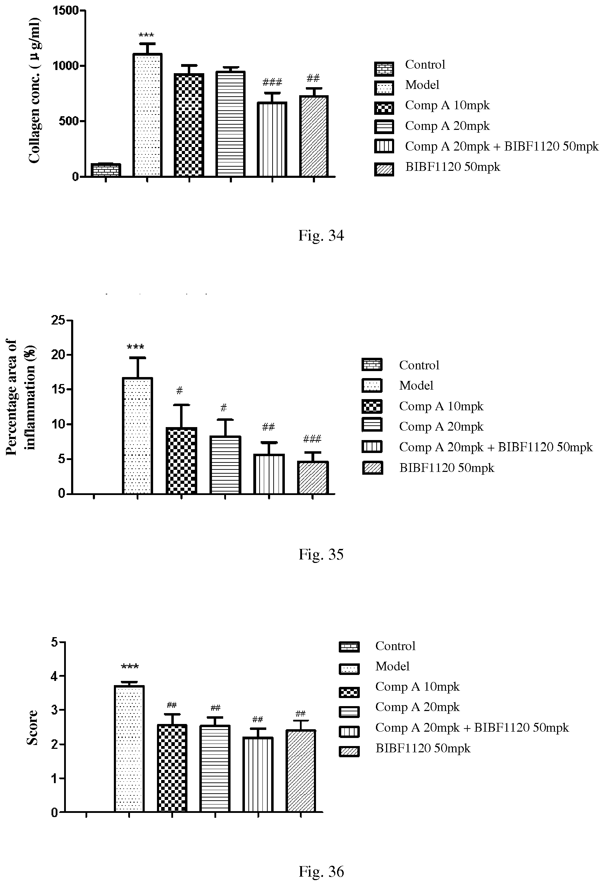

After BLM induction, the content of soluble collagen in the bronchoalveolar lavage fluid (BALF) of mice was significantly increased (p<0.001). After compound treatment, the soluble collagen content in BALF of mice was reduced, and the soluble collagen content in compound A 20mpk combined with BIBF1120 50mpk and BIBF1120 50mpk group was significantly reduced (p<0.001; P <0.01) (Fig 34).

### 4. Results of H & E staining and percentage of lung inflammation areas in lung tissue

The control group showed normal lung tissue morphology, and BLM infusion caused severe pathological changes in the lungs of mice, including alveolar tissue structure destruction and inflammatory cell infiltration, pulmonary vascular and bronchial inflammatory cell infiltration, which significantly increased the infiltration area of inflammatory cells in the lung tissue (p<0.001). After compound treatment, the area of inflammatory infiltration in the lung tissue of mice was decreased, the pathological changes were improved, and partial of lung structure returned to normal. Wherein, the inflammatory infiltration area in compound A 10mpk group and 20mpk group was significantly decreased (p < 0.05); the inflammatory area of compound A 20mpk combined with BIBF1120 50mpk and BIBF1120 50mpk group was significantly decreased (p < 0.01; p < 0.001), which was statistically significant (Table 14, Fig. 35). The efficacy of compound A combined with BIBF1120 was similar to that of BIBF1120.

**Table 14 Inflammatory infiltration area of lung tissue (n=10, Mean ± Sem)**

| Group | Pulmonary inflammatory infiltration parameters | | |
|---|---|---|---|
| | Inflammatory infiltration area (mm²) | Total lung tissue area (mm²) | Percentage of inflammatory area (%) |
| Blank control group | 0.00±0.00 | 73.52±3.46 | 0.00±0.00 |
| Modeling group | 17.63±3.63 | 104.10±4.18 | 16.63±2.96*** |
| BLM+Comp A 10mpk | 8.80±2.79 | 100.20±3.61 | 9.42±3.35^{#} |
| BLM+Comp A 20mpk | 7.85±2.28 | 96.41±3.28 | 8.23±2.40^{#} |
| BLM+Comp A 20mpk+BIBF1120 50mpk | 5.43±1.87 | 89.41±3.62 | 5.67±1.74^{##} |
| BLM+BIBF1120 50mpk | 4.33±1.39 | 87.02±4.05 | 4.67±1.32^{###} |

| | | | |
|---|---|---|---|
| Note: Analysis by Mann-Whitney nonparametric test: *** p<0.001 vs. Blank control group; ^{#} <0.05, ^{##} p<0.01, ^{###} p<0.001 vs. BLM model group. | | | |

### 5. The analysis results of Masson staining and pulmonary fibrosis

The blank control group showed normal lung tissue morphology and some normal collagen protein around the pulmonary duct. After induction of BLM, pulmonary fibrosis was very significant, including diffuse fibrosis, thickening of alveolar septum, intracavitary fibrosis and disappearance of normal alveolar structure. After treatment, some lung tissues were improved, alveolar structure returned to normal, and compound A 10mpk and 20mpk, Compound A 20mpk combined with BIBF1120 50mpk, and BIBF1120 50mpk group significantly decreased the modified pathological Ashcroft score of pulmonary fibrosis injury (p < 0. 01) (Fig 36).

### 6. immunohistochemical results of α-SMA in lung tissue

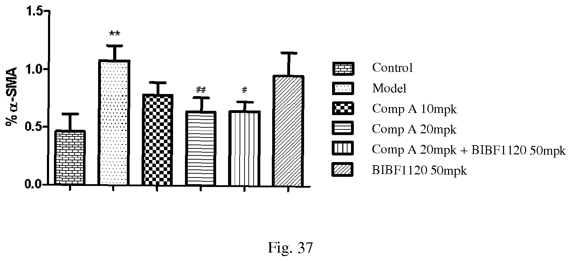

In the control group, there was almost no α-SMA immunohistochemical staining in the normal lung tissue except around the blood vessels and bronchi. After BLM induction, pulmonary fibrosis and immunohistochemical staining with α-SMA were significantly increased. Immunohistochemical staining of α-SMA was significantly decreased in compound A 20mpk group and compound A 20mpk combined with BIBF1120 50mpk group (p<0.01; p <0.05), with statistical significance (Table 15, Fig 37). Among them, compound A 20mpk group showed significant efficacy, while BIBF1120 group showed no significant efficacy.

**Table 15 Positive results of α-SMA immunohistochemical staining in lung tissue (n=10, Mean ± Sem)**

| Group | Blank control group | Model group | A 10mpk | A 20mpk | A 20mpk +BIBF1120 | BIBF1120 |
|---|---|---|---|---|---|---|
| Percentage of positiveα-SMA (%) | 0.48±0.16 | 1.08±0.13** | 0.78±0.11 | 0.64±0.12^{##} | 0.65±0.08^{#} | 0.96±0.20 |

| | | | | | | |
|---|---|---|---|---|---|---|
| Note: Analysis by Mann-Whitney nonparametric test: ** p<0.01 vs. Blank control group; ^{#}p<0.05, ^{##}p<0.01 vs. Model group. | | | | | | |

### Conclusion:

After BLM induction, the body weight of mice in the model group was significantly reduced, and the content of soluble collagen in the bronchoalveolar lavage solution of mice was significantly increased, resulting in a significant increase in the infiltration area of inflammatory cells in the lung tissue of mice, which further led to a large amount of collagen deposition and caused severe fibrosis. After treatment, various indexes of mice were improved, among which compound A 20mpk combined with BIBF1120 50mpk and BIBF1120 50mpk significantly reduced the content of soluble collagen in alveolar lavage fluid. Compound A 10mpk and 20mpk significantly reduced the percentage of inflammatory infiltration area. Compound A 20mpk combined with BIBF1120 50mpk and BIBF1120 50mpk significantly reduced the percentage of inflammatory infiltration area. The efficacy of compound A combined with BIBF1120 group was similar to that of BIBF1120 group. Compound A 10mpk, compound A 20mpk, compound A 20mpk combined with BIBF1120 50mpk and BIBF1120 50mpk significantly reduced the modified pathological Ashcroft score of pulmonary fibrosis injury. Compound A 20mpk group and compound A 20mpk combined with BIBF1120 50mpk also significantly reduced the percentage of α-SMA immunohistochemical staining. Compound A showed significant efficacy, while BIBF1120 showed no significant efficacy.

To sum up, compound A alone showed significant inhibitory effect on bleomycin-induced lung inflammation and fibrosis. Compound A combined with BIBF1120 and compound BIBF1120 alone also showed good anti-inflammatory and anti-pulmonary fibrosis effects.

Having now fully described this invention, it will be understood by those of ordinary skill in the art that the same can be performed within a wide and equivalent range of conditions, formulations and other parameters without affecting the scope of the disclosure or any embodiment thereof. All patents, patent applications and publications cited herein are fully incorporated by reference herein in their entirety.

## Claims

1. A method for treating or preventing fibrosis diseases, including administering a subject in need thereof with an effective amount of a hedgehog pathway inhibitor, wherein said hedgehog pathway inhibitor is a compound represented by Formula (I) or a pharmaceutically acceptable salt or prodrug thereof: 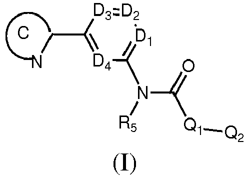 wherein: the C cyclic group is an optionally substituted N-containing heteroaryl;
D₁ is N or CR₆; D₂ is N or CR₇; D₃ is N or CR₈; D₄ is N or CR₉;
Q₁ and Q₂ are independently optionally substituted aryl, heteroaryl, a carbocyclic group or heterocyclic group;
R6-R9 are independently hydrogen, halo, optionally substituted amino, alkoxy, C₁₋₁₀ alkyl, C₃₋₈ cycloalkyl, haloalkyl, aryl, a carbocyclic group, a heterocyclic group, heteroaryl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, hydroxyalkoxy, aminoalkyl, aminoalkoxy, carboxyalkyl, carboxyalkoxy, nitro, cyano, acylamino, aminocarbonyl, hydroxy, thiol, acyloxy, azido, carboxy, hydroxyacylamino, alkylsulfonyl, aminosulfonyl, dialkylaminosulfonyl, alkylsulfinyl, or alkylthiol;
R₅ is hydrogen or C₁₋₁₀ alkyl, or R₅ is taken together with N atom to which it is attached, and carbon of C(=O), and an atom of Q₁ to form a heterocyclic group.

2. The method of claim 1, wherein the hedgehog pathway inhibitor is a compound represented by Formula (II) or a pharmaceutically acceptable salt thereof: 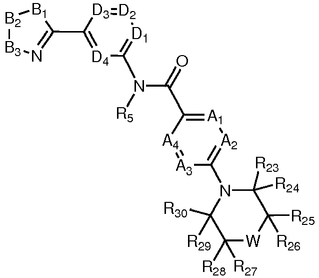
wherein: B₁ is NR₁₄; B₂ is CR₁₁; B₃ is CR₁₂;
R₁₁ and R₁₂ are independently hydrogen, C₁₋₆ alkyl, C₃₋₈ cycloalkyl, thienyl and thiazolyl optionally substituted by 1, 2 or 3 groups of C₁₋₆ alkyl and halo, pyrrolyl optionally substituted by 1-4 groups of C₁₋₆ alkyl, or furyl optionally substituted by 1, 2 or 3 groups of C₁₋₆ alkyl, or, R₁₁ and R₁₂ are taken together with the C atom to which they are attached to form phenyl, pyridyl or thienyl, which is optionally substituted by 1 or 2 groups of halo and C₁₋₆ alkyl; R₁₄ is H;
D₁ is N or CR₆, D₂ is N or CR₇, D₃ is N or CR₈, D₄ is N or CR₉, provided that, (1) D₁ is CR₆, D₂ is CR₇, D₃ is CR₈, and D₄ is CR₉, or (2) one of D₁-D₄ is N; wherein, R₆-R₉ are independently selected from the group consisting of H, halo and C₁₋₆ alkyl;
R₅ is H;
A₁ is N or CR₁, A₂ is N or CR₂, A₃ is N or CR₃, A₄ is N or CR₄; provided that, (1) A₁ is CR₁, A₂ is CR₂, A₃ is CR₃, and A₄ is CR₄, or (2) one of A₁-A₄ is N; wherein, R₁-R₄ are independently H, halo, C₁₋₆ alkyl or C₁₋₆ haloalkyl;
W is NR₃₃;
R₂₃, R₂₄, R₂₉ and R₃₀ are independently H;
R₂₅ and R₂₆ are each independently H or C₁₋₆ alkyl, provided that at least one of R₂₅ and R₂₆ is C₁₋₆ alkyl;
R₂₇ and R₂₈ are each independently H or C₁₋₆ alkyl, provided that at least one of R₂₇ and R₂₈ is C₁₋₆ alkyl; and
R₃₃ is C₁₋₆ alkyl.

3. The method of claim 2, wherein
D₁ is CR₆, D₂ is CR₇, D₃ is CR₈, and D₄ is CR₉; and/or
A₁ is CR₁, A₂ is CR₂, A₃ is CR₃, and A₄ is CR₄.

4. The method of claim 2 or 3, wherein
R₁-R₄ are independently H, halo, C₁₋₃ alkyl and C₁₋₃ haloalkyl; and/or
R₆-R₉ are independently H, halo or C₁₋₃ alkyl; and/or
R₁₁ and R₁₂ are each independently hydrogen, C₁₋₆ alkyl, C₃₋₈ cycloalkyl, thienyl and thiazolyl optionally substituted by 1, 2 or 3 groups of C₁₋₆ alkyl and halo, pyrrolyl optionally substituted by 1-4 groups of C₁₋₆ alkyl, or furyl optionally substituted by 1, 2 or 3 groups of C₁₋₆ alkyl; and/or
R₂₅ and R₂₇ are H; and/or
R₂₆, R₂₈ and R₃₃ are independently C₁₋₃ alkyl.

5. The method of claim 2, wherein
D₁ is CR₆, D₂ is CR₇, D₃ is CR₈, and D₄ is CR₉;
A₁ is CR₁, A₂ is CR₂, A₃ is CR₃, and A₄ is CR₄;
R₁₁ is H, C₁₋₆ alkyl or C₃₋₈ cycloalkyl; R₁₂ is C₁₋₆ alkyl or C₃₋₈ cycloalkyl; or R₁₁ is C₁₋₆ alkyl or C₃₋₈ cycloalkyl; R₁₂ is H, C₁₋₆ alkyl or C₃₋₈ cycloalkyl;
R₂₅ and R₂₇ are H; and
R₂₆, R₂₈ and R₃₃ are independently C₁₋₃ alkyl.

6. The method of claim 2, wherein
R₁₁ and R₁₂ are independently hydrogen, C₁₋₆ alkyl, C₃₋₈ cycloalkyl, thienyl and thiazolyl optionally substituted by 1, 2 or 3 groups of C₁₋₆ alkyl and halo, pyrrolyl optionally substituted by 1-4 groups of C₁₋₆ alkyl, or furyl optionally substituted by 1, 2 or 3 groups of C₁₋₆ alkyl;
D₁ is CR₆, D₂ is CR₇, D₃ is CR₈, and D₄ is CR₉; and
A₁ is CR₁, A₂ is CR₂, A₃ is CR₃, and A₄ is CR₄;

7. The method of claim 1, wherein said hedegehog pathway inhibitor is selected from:
N-(3-(5-(thiophen-2-yl)-1H-imidazol-2-yl)-4-chlorophenyl)-2-methyl-4-((3S,5R)-3,4,5-trimethylpiperazin-1-yl)benzamide;
N-(3-(5-(thiophen-2-yl)-1H-imidazol-2-yl)-4-chlorophenyl)-2-chloro-4-((3S,5R)-3,4,5-trimethylpiperazin-1-yl)benzamide;
N-(5-(1H-benzo[d]imidazol-2-yl)-6-chloropyridin-3-yl)-6-((3S,5R)-3,4,5-trimethylpiperazin-1-yl)nicotinamide;
N-(5-(1H-benzo[d]imidazol-2-yl)-6-chloropyridin-3-yl)-2-methyl-4-((3S,5R)-3,4,5-trimethylpiperazin-1-yl)benzamide;
N-(5-(1H-benzo[d]imidazol-2-yl)-6-methylpyridin-3-yl)-2-methyl-4-((3S,5R)-3,4,5-trimethylpiperazin-1-yl)benzamide;
N-(5-(1H-benzo[d]imidazol-2-yl)-6-methylpyridin-3-yl)-6-((3S,5R)-3,4,5-trimethylpiperazin-1-yl)nicotinamide;
N-(3-(1H-benzo[d]imidazol-2-yl)-4-chlorophenyl)-2-methyl-4-((3S,5R)-3,4,5-trimethylpiperazin-1-yl)benzamide;
N-(3-(1H-benzo[d]imidazol-2-yl)-4-chlorophenyl)-2-chloro-4-((3S,5R)-3,4,5-trimethylpiperazin-1-yl)benzamide;
N-(3-(1H-benzo[d]imidazol-2-yl)-4-chlorophenyl)-2-fluoro-4-((3S,5R)-3,4,5-trimethylpiperazin-1-yl)benzamide;
N-(3-(6-chloro-1H-benzo[d]imidazol-2-yl)-4-chlorophenyl)-2-chloro-4-((3S,5R)-3,4,5-trimethylpiperazin-1-yl)benzamide;
N-(3-(6-chloro-1H-benzo[d]imidazol-2-yl)-4-chlorophenyl)-2-methyl-4-((3S,5R)-3,4,5-trimethylpiperazin-1-yl)benzamide;
N-(3-(6-fluoro-1H-benzo[d]imidazol-2-yl)-4-chlorophenyl)-2-methyl-4-((3S,SR)-3,4,5-trimethylpiperazin-1-yl)benzamide;
N-(3-(6-fluoro-1H-benzo[d]imidazol-2-yl)-4-chlorophenyl)-2-chloro-4-((3S,SR)-3,4,5-trimethylpiperazin-1-yl)benzamide;
N-(3-(1H-benzo[d]imidazol-2-yl)-4-chlorophenyl)-2-methyl-4-((3S,5R)-4-ethyl-3,5-dimethylpiperazin-1-yl)benzamide;
N-(3-(1H-benzo[d]imidazol-2-yl)-4-chlorophenyl)-2-methyl-4-((3S,5R)-4-isopropyl-3,5-dimethylpiperazin-1-yl)benzamide;
N-(3-(1H-benzo[d]imidazol-2-yl)-4-methylphenyl)-2-chloro-4-((3S,5R)-3,4,5-trimethylpiperazin-1-yl)benzamide;
N-(3-(1H-benzo[d]imidazol-2-yl)-4-chlorophenyl)-6-((3S,5R)-3,4,5-trimethylpiperazin-1-yl)nicotinamide;
N-(3-(1H-benzo[d]imidazol-2-yl)-4-chlorophenyl)-6-((3S,5R)-4-ethyl-3,5-dimethylpiperazin-1-yl)nicotinamide;
N-(3-(1H-benzo[d]imidazol-2-yl)-4-chlorophenyl)-4-methyl-6-((3S,5R)-3,4,5-trimethylpiperazin-1-yl)nicotinamide;
N-(3-(1-methyl-1H-benzo[d]imidazol-2-yl)-4-chlorophenyl)-2-chloro-4-((3S,5R)-3,4,5-trimethylpiperazin-1-yl)benzamide;
N-(3-(6-methyl-1H-benzo[d]imidazol-2-yl)-4-chlorophenyl)-2-chloro-4-((3S,5R)-3,4,5-trimethylpiperazin-1-yl)benzamide;
N-(3-(6-methyl-1H-benzo[d]imidazol-2-yl)-4-chlorophenyl)-2-methyl-4-((3S,5R)-3,4,5-trimethylpiperazin-1-yl)benzamide;
N-(3-(6-fluoro-1H-benzo[d]imidazol-2-yl)-4-chlorophenyl)-2-chloro-4-((3S,5R)-4-ethyl-3,5-dimethylpiperazin-1-yl)benzamide;
N-(3-(6-fluoro-1H-benzo[d]imidazol-2-yl)-4-methylphenyl)-2-chloro-4-((3S,5R)-3,4,5-trimethylpiperazin-1-yl)benzamide;
N-(3-(1H-benzo[d]imidazol-2-yl)-4-chlorophenyl)-2-methyl-6-((3S,5R)-3,4,5-trimethylpiperazin-1-yl)nicotinamide;
N-(3-(1H-benzo[d]imidazol-2-yl)-4-chlorophenyl)-2-chloro-6-((3S,5R)-3,4,5-trimethylpiperazin-1-yl)nicotinamide;
N-(3-(1H-benzo[d]imidazol-2-yl)-4-chlorophenyl)-4-((3S,5R)-3,4,5-trimethylpiperazin-1-yl)benzamide;
N-(3-(1H-benzo[d]imidazol-2-yl)-4-chlorophenyl)-2-trifluoromethyl-4-((3S,5R)-3,4,5-trimethylpiperazin-1-yl)benzamide;
N-(3-(1H-benzo[d]imidazol-2-yl)-4-chlorophenyl)-3-chloro-4-((3S,5R)-3,4,5-trimethylpiperazin-1-yl)benzamide;
N-(3-(1H-benzo[d]imidazol-2-yl)-4-chlorophenyl)-3-methyl-4-((3S,5R)-3,4,5-trimethylpiperazin-1-yl)benzamide;
N-(3-(1H-benzo[d]imidazol-2-yl)-4-chlorophenyl)-2-methyl-3-fluoro-4-((3S,5R)-3,4,5-trimethylpiperazin-1-yl)benzamide;
N-(3-(1H-benzo[d]imidazol-2-yl)-4-chlorophenyl)-2-chloro-5-fluoro-4-((3S,5R)-3,4,5-trimethylpiperazin-1-yl)benzamide;
N-(3-(1H-benzo[d]imidazol-2-yl)-4-chlorophenyl)-2-chloro-4-((3S,5R)-4-ethyl-3,5-dimethylpiperazin-1-yl)benzamide;
N-(3-(1H-benzo[d]imidazol-2-yl)-4-chlorophenyl)-2-chloro-4-((3S,5R)-4-isopropyl-3,5-dimethylpiperazin-1-yl)benzamide;
N-(5-(1H-benzo[d]imidazol-2-yl)-6-chloropyridin-3-yl)-2-chloro-4-((3S,5R)-3,4,5-trimethylpiperazin-1-yl)benzamide;
N-(5-(6-fluoro-1H-benzo[d]imidazol-2-yl)-6-chloropyridin-3-yl)-2-chloro-4-((3S,5R)-3,4,5-trimethylpiperazin-1-yl)benzamide;
N-(5-(6-chloro-1H-benzo[d]imidazol-2-yl)-6-chloropyridin-3-yl)-2-chloro-4-((3S,5R)-3,4,5-trimethylpiperazin-1-yl)benzamide;
N-(3-(6-fluoro-1H-benzo[d]imidazol-2-yl)-4-chlorophenyl)-2-chloro-4-((3S,5R)-3,4,5-trimethylpiperazin-1-yl)benzamide dihydrochloride;
N-(3-(1H-benzo[d]imidazol-2-yl)-4-chlorophenyl)-2-chloro-4-((3S,5R)-3,4,5-trimethylpiperazin-1-yl)benzamide dihydrochloride;
N-(3-(1H-benzo[d]imidazol-2-yl)-4-chlorophenyl)-3-chloro-4-((3S,5R)-3,4,5-trimethylpiperazin-1-yl)benzamide dihydrochloride;
N-(3-(5-(4-methylthiophen-2-yl)-1H-imidazol-2-yl)-4-chlorophenyl)-2-methyl-4-((3S,5R)-3,4,5-trimethylpiperazin-1-yl)benzamide;
N-(3-(5-(4-methylthiophen-2-yl)-1H-imidazol-2-yl)-4-chlorophenyl)-2-chloro-4-((3S,5R)-3,4,5-trimethylpiperazin-1-yl)benzamide;
N-(3-(5-(5-chlorothiophen-2-yl)-1H-imidazol-2-yl)-4-chlorophenyl)-3-chloro-4-((3S,5R)-3,4,5-trimethylpiperazin-1-yl)benzamide;
N-(3-(5-(5-chlorothiophen-2-yl)-1H-imidazol-2-yl)-4-chlorophenyl)-2-chloro-4-((3S,5R)-3,4,5-trimethylpiperazin-1-yl)benzamide;
N-(3-(5-(thiophen-3-yl)-1H-imidazol-2-yl)-4-chlorophenyl)-2-methyl-4-((3S,5R)-3,4,5-trimethylpiperazin-1-yl)benzamide;
N-(3-(5-(thiophen-3-yl)-1H-imidazol-2-yl)-4-chlorophenyl)-2-chloro-4-((3S,5R)-3,4,5-trimethylpiperazin-1-yl)benzamide;
N-(3-(5-(thiophen-3-yl)-1H-imidazol-2-yl)-4-chlorophenyl)-2-methyl-3-fluoro-4-((3S,5R)-3,4,5-trimethylpiperazin-1-yl)benzamide;
N-(3-(5-(thiophen-3-yl)-1H-imidazol-2-yl)-4-chlorophenyl)-2-chloro-5-fluoro-4-((3S,5R)-3,4,5-trimethylpiperazin-1-yl)benzamide;
N-(3-(4-methyl-5-(thiophen-2-yl)-1H-imidazol-2-yl)-4-chlorophenyl)-2-methyl-4-((3S,5R)-3,4,5-trimethylpiperazin-1-yl)benzamide;
N-(3-(5-(furan-2-yl)-1H-imidazol-2-yl)-4-chlorophenyl)-2-chloro-4-((3S,5R)-3,4,5-trimethylpiperazin-1-yl)benzamide;
N-(3-(5-(furan-2-yl)-1H-imidazol-2-yl)-4-chlorophenyl)-2-methyl-4-((3S,5R)-3,4,5-trimethylpiperazin-1-yl)benzamide;
N-(3-(5-(furan-2-yl)-1H-imidazol-2-yl)-4-chlorophenyl)-2-trifluoromethyl-4-((3S,5R)-3,4,5-trimethylpiperazin-1-yl)benzamide;
N-(3-(5-(furan-2-yl)-1H-imidazol-2-yl)-4-chlorophenyl)-2-methyl-3-fluoro-4-((3S,5R)-3,4,5-trimethylpiperazin-1-yl)benzamide;
N-(3-(5-(furan-2-yl)-1H-imidazol-2-yl)-4-chlorophenyl)-2-chloro-5-fluoro-4-((3S,5R)-3,4,5-trimethylpiperazin-1-yl)benzamide;
N-(3-(5-(furan-2-yl)-1H-imidazol-2-yl)-4-chlorophenyl)-2-chloro-4-((3S,5R)-4-ethyl-3,5-dimethylpiperazin-1-yl)benzamide;
N-(3-(5-(5-methylfuran-2-yl)-1H-imidazol-2-yl)-4-chlorophenyl)-2-chloro-4-((3S,5R)-3,4,5-trimethylpiperazin-1-yl)benzamide;
N-(3-(5-(5-methylfuran-2-yl)-1H-imidazol-2-yl)-4-chlorophenyl)-2-methyl-4-((3S,5R)-3,4,5-trimethylpiperazin-1-yl)benzamide;
N-(3-(5-(thiazol-2-yl)-1H-imidazol-2-yl)-4-chlorophenyl)-2-chloro-4-((3S,5R)-3,4,5-trimethylpiperazin-1-yl)benzamide;
N-(3-(5-(thiazol-2-yl)-1H-imidazol-2-yl)-4-chlorophenyl)-2-methyl-4-((3S,5R)-3,4,5-trimethylpiperazin-1-yl)benzamide;
N-(3-(5-(thiophen-2-yl)-1H-imidazol-2-yl)-4-chlorophenyl)-2-trifluoromethyl-4-((3S,5R)-3,4,5-trimethylpiperazin-1-yl)benzamide;
N-(3-(5-(thiophen-2-yl)-1H-imidazol-2-yl)-4-chlorophenyl)-2-methyl-3-fluoro-4-((3S,5R)-3,4,5-trimethylpiperazin-1-yl)benzamide;
N-(3-(5-(thiophen-2-yl)-1H-imidazol-2-yl)-4-chlorophenyl)-2-chloro-5-fluoro-4-((3S,5R)-3,4,5-trimethylpiperazin-1-yl)benzamide;
N-(3-(5-(thiophen-2-yl)-1H-imidazol-2-yl)-4-chlorophenyl)-2-chloro-4-((3S,5R)-4-ethyl-3,5-dimethylpiperazin-1-yl)benzamide;
N-(3-(5-(thiophen-2-yl)-1H-imidazol-2-yl)-4-chlorophenyl)-2-chloro-4-((3S,5R)-4-isopropyl-3,5-dimethylpiperazin-1-yl)benzamide;
N-(3-(5-(1-methyl-1H-pyrrol-2-yl)-1H-imidazol-2-yl)-4-chlorophenyl)-2-methyl-4-((3S,5R)-3,4,5-trimethylpiperazin-1-yl)benzamide;
N-(3-(5-(1-methyl-1H-pyrrol-2-yl)-1H-imidazol-2-yl)-4-chlorophenyl)-2-chloro-4-((3S,5R)-3,4,5-trimethylpiperazin-1-yl)benzamide;
N-(3-(5-(thiophen-3-yl)-1H-imidazol-2-yl)-4-chlorophenyl)-2-trifluoromethyl-4-((3S,5R)-3,4,5-trimethylpiperazin-1-yl)benzamide dihydrochloride;
N-(3-(5-(thiophen-3-yl)-1H-imidazol-2-yl)-4-chlorophenyl)-2-chloro-4-((3S,5R)-4-ethyl-3,5-dimethylpiperazin-1-yl)benzamide dihydrochloride;
N-(3-(1H-imidazol-2-yl)-4-chlorophenyl)-2-methyl-4-((3S,5R)-3,4,5-trimethylpiperazin-1-yl)benzamide;
N-(3-(5-ethyl-1H-imidazol-2-yl)-4-chlorophenyl)-2-trifluoromethyl-4-((3S,5R)-3,4,5-trimethylpiperazin-1-yl)benzamide;
N-(3-(5-ethyl-1H-imidazol-2-yl)-4-chlorophenyl)-2-methyl-3-fluoro-4-((3S,5R)-3,4,5-trimethylpiperazin-1-yl)benzamide;
N-(3-(5-ethyl-1H-imidazol-2-yl)-4-chlorophenyl)-2-chloro-5-fluoro-4-((3S,5R)-3,4,5-trimethylpiperazin-1-yl)benzamide;
N-(3-(5-ethyl-1H-imidazol-2-yl)-4-chlorophenyl)-2-chloro-4-((3S,5R)-4-ethyl-3,5-dimethylpiperazin-1-yl)benzamide;
N-(3-(5-isopropyl-1H-imidazol-2-yl)-4-chlorophenyl)-2-methyl-4-((3S,5R)-3,4,5-trimethylpiperazin-1-yl)benzamide;
N-(3-(5-isopropyl-1H-imidazol-2-yl)-4-chlorophenyl)-2-trifluoromethyl-4-((3S,5R)-3,4,5-trimethylpiperazin-1-yl)benzamide;
N-(3-(5-propyl-1H-imidazol-2-yl)-4-chlorophenyl)-2-methyl-4-((3S,5R)-3,4,5-trimethylpiperazin-1-yl)benzamide;
N-(3-(5-propyl-1H-imidazol-2-yl)-4-chlorophenyl)-2-chloro-4-((3S,5R)-3,4,5-trimethylpiperazin-1-yl)benzamide;
N-(3-(5-tert-butyl-lH-imidazol-2-yl)-4-chlorophenyl)-2-methyl-4-((3S,5R)-3,4,5-trimethylpiperazin-1-yl)benzamide;
N-(3-(5-tert-butyl-1H-imidazol-2-yl)-4-chlorophenyl)-2-chloro-4-((3S,5R)-3,4,5-trimethylpiperazin-1-yl)benzamide;
N-(3-(5-cyclopropyl-1H-imidazol-2-yl)-4-chlorophenyl)-2-chloro-4-((3S,5R)-3,4,5-trimethylpiperazin-1-yl)benzamide;
N-(3-(5-cyclopropyl-1H-imidazol-2-yl)-4-chlorophenyl)-2-methyl-4-((3S,5R)-3,4,5-trimethylpiperazin-1-yl)benzamide;
N-(3-(5-cyclopropyl-1H-imidazol-2-yl)-4-chlorophenyl)-2-trifluoromethyl-4-((3S,5R)-3,4,5-trimethylpiperazin-1-yl)benzamide;
N-(3-(5-cyclopropyl-1H-imidazol-2-yl)-4-chlorophenyl)-2-methyl-3-fluoro-4-((3S,5R)-3,4,5-trimethylpiperazin-1-yl)benzamide;
N-(3-(5-cyclobutyl-1H-imidazol-2-yl)-4-chlorophenyl)-2-chloro-4-((3S,5R)-3,4,5-trimethylpiperazin-1-yl)benzamide;
N-(3-(5-cyclobutyl-1H-imidazol-2-yl)-4-chlorophenyl)-2-methyl-4-((3S,5R)-3,4,5-trimethylpiperazin-1-yl)benzamide;
N-(3-(5-cyclopentyl-1H-imidazol-2-yl)-4-chlorophenyl)-2-chloro-4-((3S,5R)-3,4,5-trimethylpiperazin-1-yl)benzamide;
N-(3-(5-cyclopentyl-lH-imidazol-2-yl)-4-chlorophenyl)-2-methyl-4-((3S,5R)-3,4,5-trimethylpiperazin-1-yl)benzamide;
N-(3-(5-cyclohexyl-1H-imidazol-2-yl)-4-chlorophenyl)-2-chloro-4-((3S,5R)-3,4,5-trimethylpiperazin-1-yl)benzamide;
N-(3-(5-cyclohexyl-1H-imidazol-2-yl)-4-chlorophenyl)-2-methyl-4-((3S,5R)-3,4,5-trimethylpiperazin-1-yl)benzamide;
N-(3-(5-methyl-1H-imidazol-2-yl)-4-chlorophenyl)-2-methyl-4-((3S,5R)-3,4,5-trimethylpiperazin-1-yl)benzamide dihydrochloride;
N-(3-(5-ethyl-1H-imidazol-2-yl)-4-chlorophenyl)-2-methyl-4-((3S,5R)-3,4,5-trimethylpiperazin-1-yl)benzamide dihydrochloride;
N-(3-(5-ethyl-1H-imidazol-2-yl)-4-chlorophenyl)-2-chloro-4-((3S,5R)-3,4,5-trimethylpiperazin-1-yl)benzamide dihydrochloride;
N-(3-(5-isopropyl-1H-imidazol-2-yl)-4-chlorophenyl)-2-chloro-4-((3S,5R)-3,4,5-trimethylpiperazin-1-yl)benzamide dihydrochloride;
N-(3-(5-isopropyl-1H-imidazol-2-yl)-4-chlorophenyl)-2-methyl-3-fluoro-4-((3S,5R)-3,4,5-trimethylpiperazin-1-yl)benzamide dihydrochloride;
N-(3-(5-isopropyl-1H-imidazol-2-yl)-4-chlorophenyl)-2-chloro-5-fluoro-4-((3S,5R)-3,4,5-trimethylpiperazin-1-yl)benzamide dihydrochloride;
N-(3-(5-isopropyl-1H-imidazol-2-yl)-4-chlorophenyl)-2-chloro-4-((3S,5R)-4-ethyl-3,5-dimethylpiperazin- 1-yl)benzamide dihydrochloride;
N-(3-(5-cyclopropyl-1H-imidazol-2-yl)-4-chlorophenyl)-2-chloro-5-fluoro-4-((3S,5R)-3,4,5-trimethylpiperazin-1-yl)benzamide dihydrochloride;
N-(3-(5-cyclopropyl-1H-imidazol-2-yl)-4-chlorophenyl)-2-chloro-4-((3S,5R)-4-ethyl-3,5-dimethylpiperazin-1-yl)benzamide dihydrochloride;
N-(3-(3H-imidazo[4,5-b]pyridin-2-yl)-4-chlorophenyl)-2-chloro-4-((3S,5R)-3,4,5-trimethylpiperazin-1-yl)benzamide;
N-(3-(3H-imidazo[4,5-c]pyridin-2-yl)-4-chlorophenyl)-2-chloro-4-((3S,5R)-3,4,5-trimethylpiperazin-1-yl)benzamide;
N-(3-(3H-imidazo[4,5-c]pyridin-2-yl)-4-chlorophenyl)-2-methyl-4-((3S,5R)-3,4,5-trimethylpiperazin-1-yl)benzamide;
N-(3-(1H-thieno[3,4-d]imidazol-2-yl)-4-chlorophenyl)-2-methyl-4-((3S,5R)-3,4,5-trimethylpiperazin-1-yl)benzamide;
N-(3-(1H-thieno[3,4-d]imidazol-2-yl)-4-chlorophenyl)-2-chloro-4-((3S,5R)-3,4,5-trimethylpiperazin-1-yl)benzamide;
N-(3-(1H-thieno[3,4-d]imidazol-2-yl)-4-chlorophenyl)-2-trifluoromethyl-4-((3S,5R)-3,4,5-trimethylpiperazin-1-yl)benzamide;
N-(3-(1H-thieno[3,4-d]imidazol-2-yl)-4-chlorophenyl)-2-methyl-3-fluoro-4-((3S,5R)-3,4,5-trimethylpiperazin-1-yl)benzamide;
N-(3-(1H-thieno[3,4-d]imidazol-2-yl)-4-chlorophenyl)-2-chloro-5-fluoro-4-((3S,5R)-3,4,5-trimethylpiperazin-1-yl)benzamide;
N-(3-(1H-thieno[3,4-d]imidazol-2-yl)-4-chlorophenyl)-2-chloro-4-((3S,5R)-4-ethyl-3,5-dimethylpiperazin-1-yl)benzamide;
or a pharmaceutically acceptable salt or prodrug thereof.

8. The method of any of claims 1-7, wherein the hedgehog pathway inhibitors are administered orally.

9. The method of any of claims 1-8, the hedgehog pathway inhibitor is used as a pharmaceutical composition including a medicinal carrier.

10. An hedgehog pathway inhibitor for treatment or prevention of a fibrosis disease, or use of hedgehog pathway inhibitors for manufacture of a medicament for treatment or prevention of fibrosis disease, wherein said hedgehog pathway inhibitor is as described in any of claims 1-7;
preferably, the fibrosis is pulmonary fibrosis, for example, pulmonary fibrosis related with an asbestosis, a cystic fibrosis, an infection (e.g., pneumonia), exposure to an environmental allergen (e.g., coal dust, asbestos, smoking, diesel emissions, ozone, particles from industrial emissions), lung transplantation, autoimmune disease (e.g. scleroderma), or drug-induced pulmonary fibrosis, preferably idiopathic pulmonary fibrosis; preferably, the fibrosis is liver fibrosis, including chronic hepatitis B, hepatitis C, nonalcoholic steatohepatitis, alcoholic liver disease, metabolic liver disease (for example, Wilson's disease, hemochromatosis), bile duct obstruction (congenital or acquired) or liver disease accompanied with unexplained fibrosis.
